# EUROPÄISCHE PATENTSCHRIFT

(11) **EP 0 623 604 B1**
(45) Veröffentlichungstag und Bekanntmachung des Hinweises auf die Patenterteilung: **08.08.2001**
(21) Anmeldenummer: 94106122.8
(22) Anmeldetag: 20.04.1994
(51) Int. Cl.: C07D 285/08, C07D 271/06, C07D 417/12, A01N 43/82

(54) **Oxa(Thia)-diazol-oxy-phenyl-acrylate als Schädlingsbekämpfungsmittel**
Oxa(Thia)-diazol-oxyphenylacrylates as pesticides
Oxa(thia)-diazolyl-oxy-phényl acrylates comme pesticides

(30) Priorität: 03.05.1993 DE 4314501; 02.12.1993 DE 4341066
(43) Veröffentlichungstag der Anmeldung: 09.11.1994
(73) Patentinhaber: BAYER AG, 51368 Leverkusen (DE)
(72) Erfinder: Gerdes, Peter, Dr., D-52080 Aachen (DE); Gayer, Herbert, Dr., D-40789 Monheim (DE); Heinemann, Ulrich, Dr., D-42799 Leichlingen (DE); Dehne, Heinz-Wilhelm, Dr., D-40789 Monheim (DE); Drewes, Mark Wilhelm, Dr., D-40764 Langenfeld (DE); Dutzmann, Stefan, Dr., D-40721 Hilden (DE)

(56) Entgegenhaltungen:
- EP-A- 0 256 667
- EP-A- 0 299 694
- EP-A- 0 393 861
- EP-A- 0 405 782
- EP-A- 0 468 695
- WO-A-94/10150
- WO-A-94/10159

## Beschreibung

Die Erfindung betrifft neue Oxa(Thia)-diazol-oxy-phenylacrylate, ein Verfahren zu ihrer Herstellung und ihre Verwendung als Schädlingsbekämpfungsmittel.

Es ist bekannt, daß bestimmte substituierte 3-Methoxy-acrylsäureester fungizide Eigenschaften besitzen (vergl. z.B. EP 178 826, EP 256 667 und EP 383 117). In WO 94/10159 werden 3-Methoxy-acrylsäureester mit füngiziden Eigenschaften beschrieben, in denen ein Oxa- oder ein Thiadiazolrest mit der Methyl 2-Phenyl-3-methoxyacrylatgruppe verknüpft ist. In WO 95/05368 werden Thia- und Oxadiazolderivate mit fungiziden Eigenschaften beschrieben. Die Wirksamkeit dieser vorbekannten Verbindungen ist jedoch insbesondere bei niedrigen Aufwandmengen und Konzentrationen nicht in allen Anwendungsgebieten völlig zufriedenstellend.

Es wurden neue Oxa(Thia)-diazol-oxy-phenylacrylate der allgemeinen Formel (I), 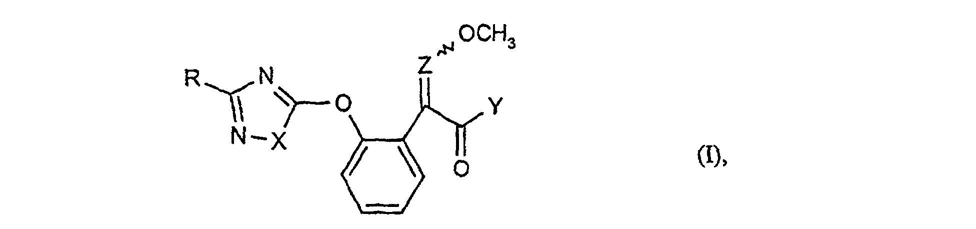 in welcher
- Y: für OCH₃ oder NHCH₃ steht,
- Z: für =CH- oder =N- steht,
- R: für Fluor, Chlor, Brom, Methyl, Ethyl, n-, i-Propyl, n-, i-, s-, t-Butyl, C₁-C₂-Halogenalkyl mit 1 bis 5 gleichen oder verschiedenen Halogenatomen oder für einen Rest der Formel Ar-A- steht, wobei
- Ar: für gegebenenfalls einfach bis fünffach, gleich oder verschieden substituiertes Aryl mit 6 oder 10 Kohlenstoffatomen oder für gegebenenfalls einfach bis vierfach, gleich oder verschieden substituiertes und/oder benzoannelliertes Heteroaryl mit 2 bis 9 Kohlenstoffatomen und 1 bis 3 gleichen oder verschieden Heteroatomen - insbesondere Stickstoff, Sauerstoff und/oder Schwefel - steht, wobei als Substituenten jeweils infrage kommen:
Halogen, Cyano, Nitro, Hydroxy, Amino, Formyl, Carbamoyl, Thiocarbamoyl, jeweils geradkettiges oder verzweigtes Alkyl, Alkoxy, Alkylthio, Alkylsulfinyl oder Alkylsulfonyl mit jeweils 1 bis 4 Kohlenstoffatomen, jeweils geradkettiges oder verzweigtes Alkenyl oder Alkenyloxy mit jeweils 2 bis 4 Kohlenstoffatomen, jeweils geradkettiges oder verzweigtes Halogenalkyl, Halogenalkoxy, Halogenalkylthio, Halogenalkylsulfinyl oder Halogenalkylsulfonyl mit jeweils 1 bis 4 Kohlenstoffatomen und 1 bis 9 gleichen oder verschiedenen Halogenatomen, jeweils geradkettiges oder verzweigtes Halogenalkenyl oder Halogenalkenyloxy mit jeweils 2 bis 4 Kohlenstoffatomen und 1 bis 9 gleichen oder verschiedenen Halogenatomen, jeweils geradkettiges oder verzweigtes N-Alkylamino, Dialkylamino, Alkylcarbonyl, Alkylcarbonyloxy, Alkoxycarbonyl, Alkylsulfonyloxy, Hydroximinoalkyl oder Alkoximinoalkyl mit jeweils 1 bis 4 Kohlenstoffatomen in den einzelnen Alkyl- und Alkoxyteilen, jeweils gegebenenfalls einfach oder mehrfach, gleich oder verschieden durch Halogen und/oder geradkettiges oder verzweigtes Alkyl mit 1 bis 3 Kohlenstoffatomen und/oder geradkettiges oder verzweigtes Halogenalkyl mit 1 bis 3 Kohlenstoffatomen und 1 bis 7 gleichen oder verschiedenen Halogenatomen substituiertes, jeweils zweifach verknüpftes Alkylen oder Dioxyalkylen mit jeweils 1 bis 4 Kohlenstoffatomen, Cycloalkyl mit 3 bis 6 Kohlenstoffatomen, 5- bis 7-gliedriges, gesättigtes Heterocyclyl mit 4 bis 6 Kohlenstoffatomen und 1 oder 2 gleichen oder verschiedenen Heteroatomen - insbesondere Stickstoff, Sauerstoff und/oder Schwefel -, sowie jeweils gegebenenfalls im Phenylteil einfach bis dreifach, gleich oder verschieden durch Halogen und/oder geradkettiges oder verzweigtes Alkyl mit 1 bis 3 Kohlenstoffatomen und/oder geradkettiges oder verzweigtes Halogenalkyl mit 1 bis 3 Kohlenstoffatomen und 1 bis 7 gleichen oder verschiedenen Halogenatomen und/oder geradkettiges oder verzweigtes Alkoxy mit 1 bis 3 Kohlenstoffatomen und/oder geradkettiges oder verzweigtes Halogenalkoxy mit 1 bis 3 Kohlenstoffatomen und 1 bis 7 gleichen oder verschiedenen Halogenatomen substituiertes Phenyl, Phenoxy, Benzyl, Benzyloxy, Phenylethyl oder Phenylethyloxy und
- A: für Sauerstoff, Schwefel oder eine Gruppierung der Formel -S(O)-, -SO₂-, -NH-, -N(CH₃)-, -CH₂O-, -CH₂S-, -OCH₂- oder -SCH₂- steht, und
- X: für Sauerstoff oder Schwefel steht,
ausgenommen Verbindungen, in denen
- R: für Fluor, Chlor, Brom, Methyl, Ethyl, n-, i-, s-, t-Butyl, C₁-C₄-Halogenalkyl mit 1 bis 5 gleichen oder verschiedenen Halogenatomen und
- X: für S steht,
gefunden.

Die Verbindungen der Formel (I) können gegebenenfalls in Abhängigkeit von der Art der Substituenten als geometrische und/oder optische Isomere oder Isomerengemische unterschiedlicher Zusammensetzung vorliegen. Sowohl die reinen Isomeren als auch die Isomerengemische werden erfindungsgemäß beansprucht.

Weiterhin wurde gefunden daß man die neuen Oxa(Thia)-diazol-oxy-phenylacrylate der allgemeinen Formel (I), 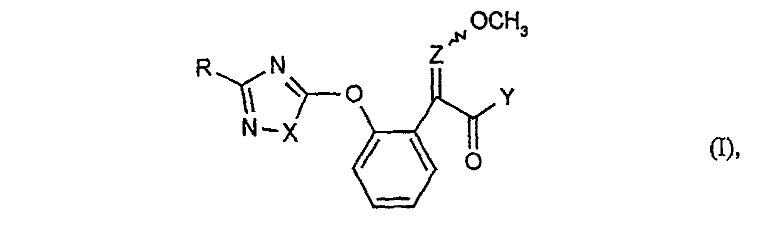 in welcher
- Y: für OCH₃ oder NHCH₃ steht,
- Z: für =CH- oder =N- steht, und
- R, Ar, A und X: die oben angegebene Bedeutung haben,
erhält, wenn man 2-Hydroxyphenyl-acrylate der Formel (II) 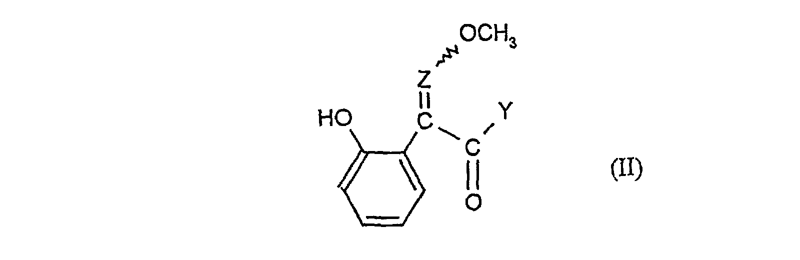 mit Oxa- bzw. Thiadiazolderivaten der Formel (III), 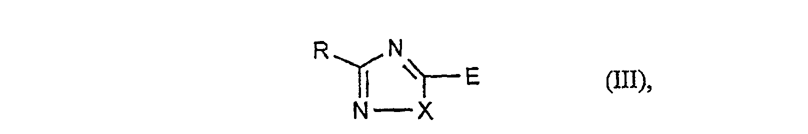 in welcher
- R und X: die oben angegebenen Bedeutungen haben und
- E: für eine elektronenanziehende Abgangsgruppe steht,
gegebenenfalls in Gegenwart eines Verdünnungsmittels und gegebenenfalls in Gegenwart eines Reaktionshilfsmittels umsetzt.

Schließlich wurde gefunden, daß die neuen Oxa(Thia)-diazol-oxy-phenylacrylate der allgemeinen Formel (I) gute Wirksamkeit gegenüber Schädlingen besitzen.

Überrraschenderweise zeigen die erfindungsgemäßen Oxa(Thia)-diazol-oxy-phenylacrylate der allgemeinen Formel (I) eine erheblich bessere Wirksamkeit gegenüber pflanzenschädigenden Mikroorganismen im Vergleich zu den aus dem Stand der Technik bekannten, substituierten 3-Methoxy-acrylsäureestem.

Die erfindungsgemäßen Oxa(Thia)-diazol-oxy-phenylacrylate sind durch die Formel (I) allgemein definiert. Bevorzugt sind Verbindungen der Formel (I), bei welchen
- R: für Chlor, Brom, Methyl, Ethyl, CF₃, CH₂F, CHF₂, CCl₃, CH₂Cl, CHCl₂, CFCl₂, CF₂Cl oder für einen Rest der Formel Ar-A- steht, wobei
- Ar: für jeweils gegebenenfalls einfach bis dreifach, gleich oder verschieden substituiertes Phenyl oder Naphthyl oder für jeweils gegebenenfalls einfach bis dreifach, gleich oder verschieden substituiertes und/oder benzoannelliertes Furanyl, Thienyl, Pyrrolyl, Oxazolyl, Thiazolyl, Isoxazolyl, Isothiazolyl, Pyrazolyl, Imidazolyl, Oxadiazolyl, Thiadiazolyl, Triazolyl, Pyridyl, Pyridazinyl, Pyrimidinyl, Pyrazinyl oder Triazinyl steht, wobei als Substituenten jeweils infrage kommen:
Fluor, Chlor, Brom, Hydroxy, Cyano, Nitro, Amino, Formyl, Carbamoyl, Thiocarbamoyl, Methyl, Ethyl, n- oder i-Propyl, n-, i-, s- oder t-Butyl, Methoxy, Ethoxy, n- oder i-Propoxy, n-, i-, s- oder t-Butoxy, Methylthio, Ethylthio, Methylsulfinyl, Methylsulfonyl, Allyl, Butenyl, Allyloxy, Butenyloxy, Trifluormethyl, Trifluormethoxy, Trifluormethylthio, Trifluormethylsulfinyl, Trifluormethylsulfonyl, Dimethylamino, Diethylamino, Acetyl, Acetoxy, Methylsulfonyloxy, Ethylsulfonyloxy, Methoxycarbonyl, Ethoxycarbonyl, Hydroximinomethyl, Hydroximinoethyl, Methoximinomethyl, Methoximinoethyl, Ethoximinomethyl, Ethoximinoethyl, Propan-1,3-diyl, Butan-1,4-diyl, Dioxyethylen, Dioxyethylen, Dioxypropylen, Difluordioxymethylen, Tetrafluordioxyethylen, Cyclopropyl, Cyclopentyl, Cyclohexyl, 1-Pyrrolidinyl, 1-Piperidinyl, 1-Perhydroazepinyl, 4-Morpholinyl oder jeweils gegebenenfalls ein- bis dreifach, gleich oder verschieden durch Fluor, Chlor, Brom, Methyl, Ethyl, Methoxy, Ethoxy, Trifluormethyl und/oder Trifluormethoxy substituiertes Phenyl, Phenoxy, Benzyl oder Benzyloxy und
- A: für Sauerstoff, Schwefel oder eine Gruppierung der Formel -S(O)-, -SO₂-, -NH-, -N(CH₃)-, -CH₂O-, -CH₂S-, -OCH₂- oder -SCH₂- steht, und
- X: für Sauerstoff oder Schwefel steht,
ausgenommen Verbindungen, in denen
- R: für Chlor, Brom, Methyl, Ethyl, CF₃, CH₂F, CHF₂, CCl₃, CH₂Cl, CHCl₂, CFCl₂, CF₂Cl und
- X: für S steht.
Von besonderem Interesse sind die Verbindungen der Formel (I) in denen X für Schwefel steht.

Von besonderem Interesse sind auch Verbindungen, in denen Z für =CH- steht und Y für -OCH₃ steht oder Z für =N- steht und Y für OCH₃ oder NHCH₃ steht.

Im einzelnen seien außer den bei den Herstellungsbeispielen genannten Verbindungen die folgenden 3-Methoxy-2-phenyl-acrylsäureester der Formel (Ia) genannt 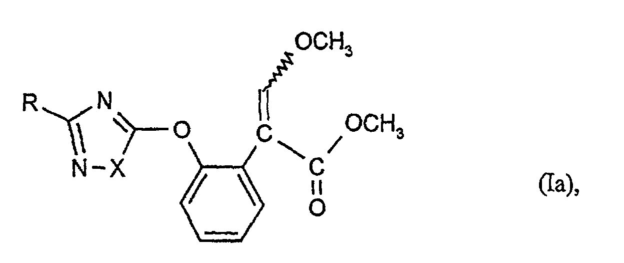 in denen X für Schwefel steht und R die in Tabelle 1 angegebenen Bedeutungen hat, sowie Verbindungen der Formel (Ia), in denen X für Sauerstoff steht und R die in Tabelle 1 angegebenen Bedeutungen hat:

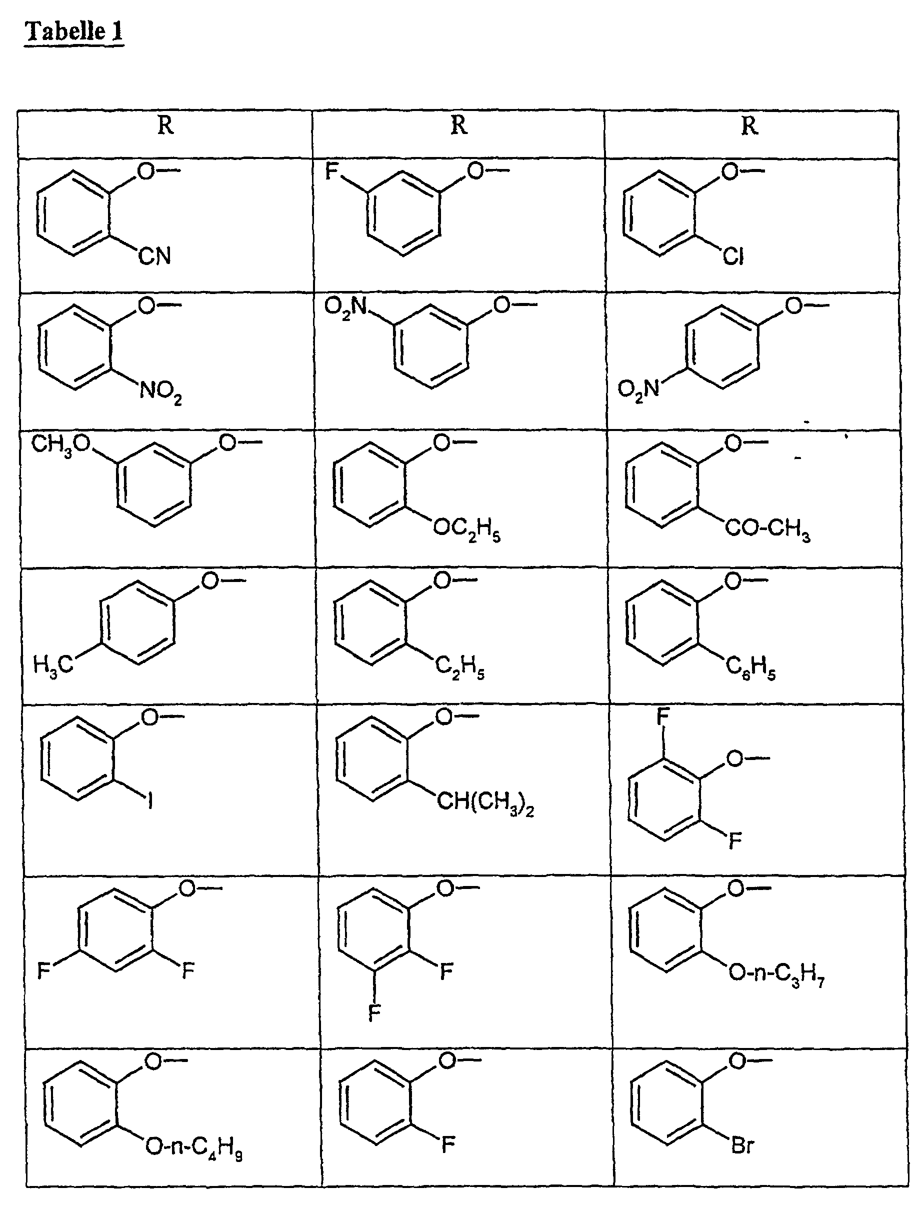

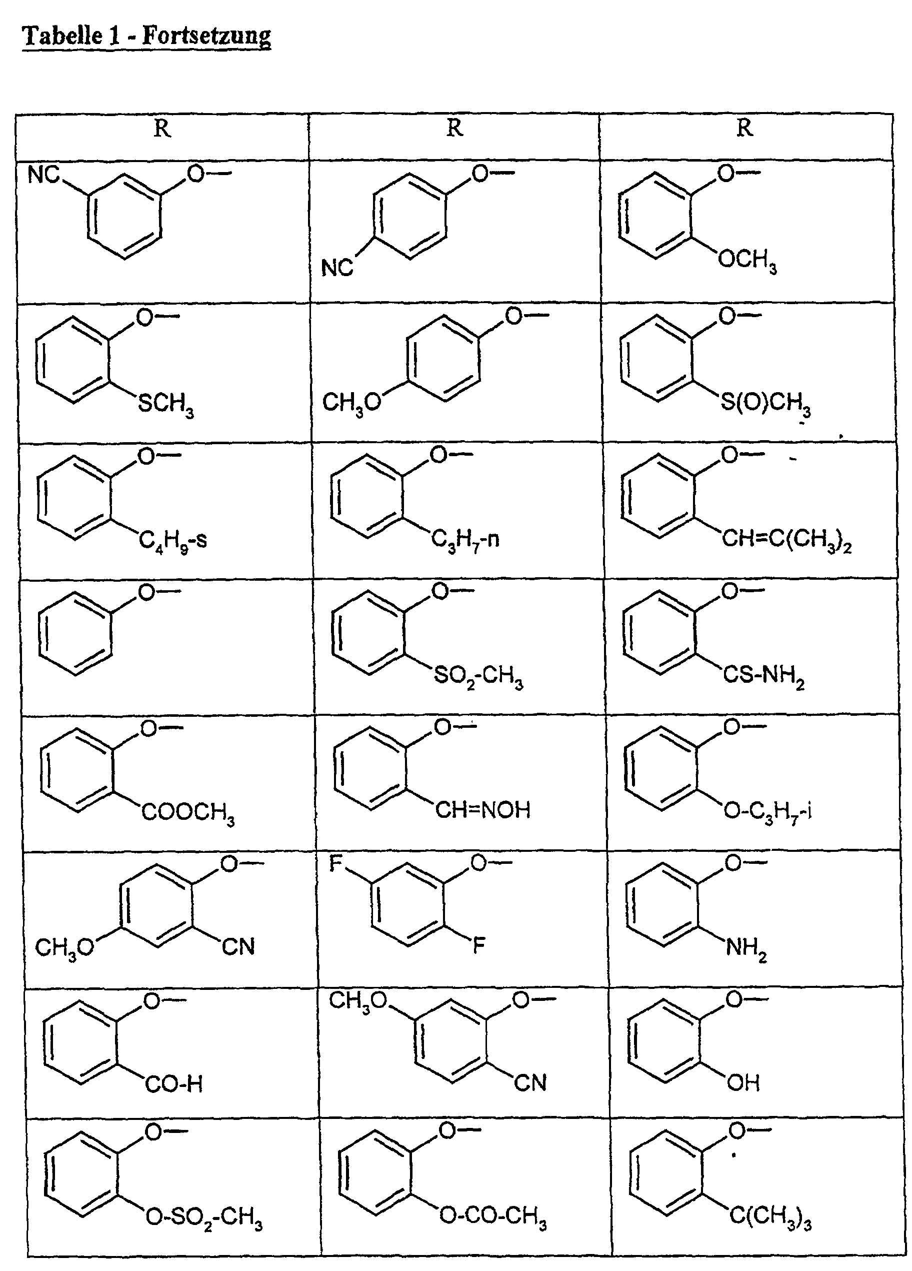

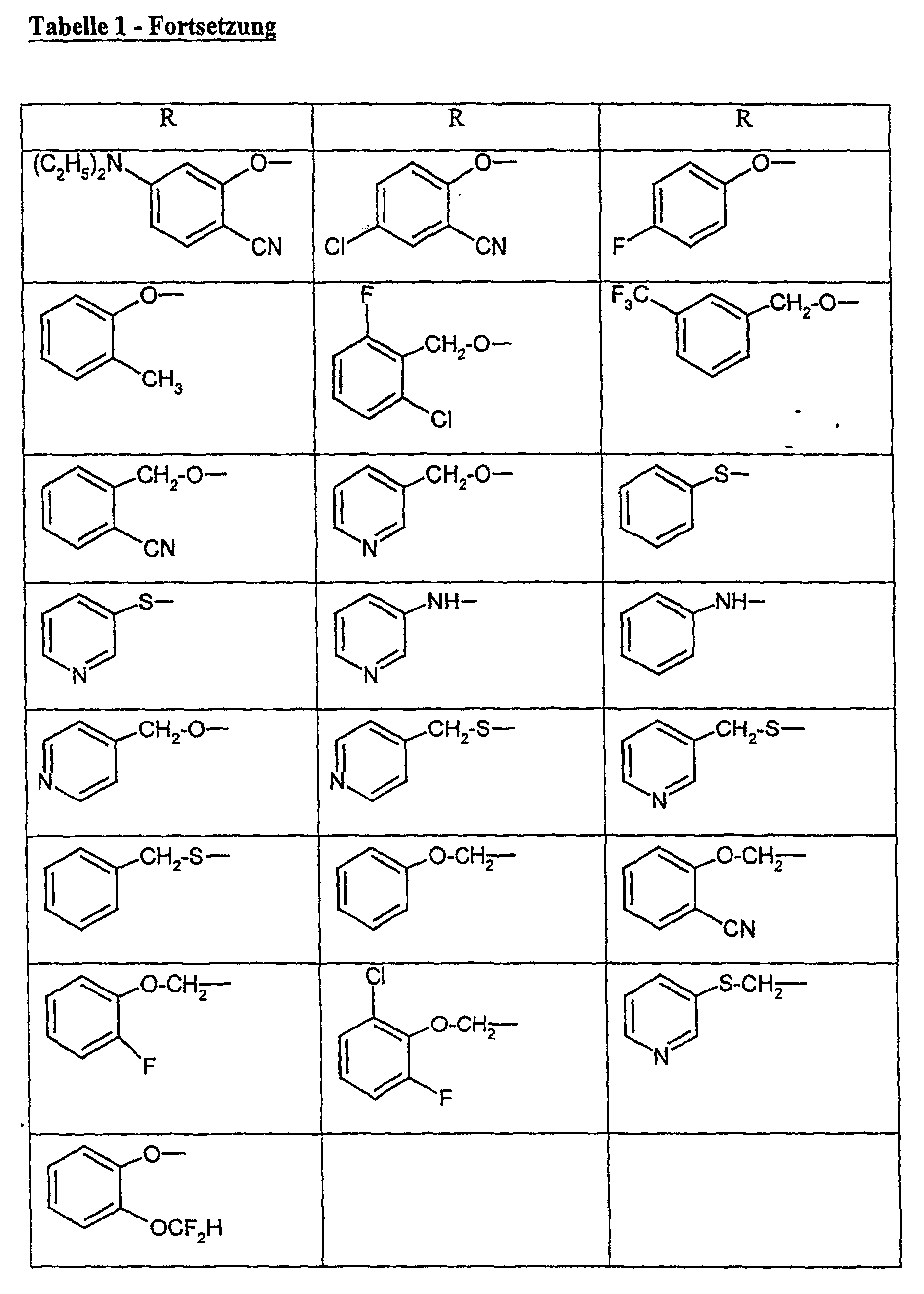

Desweiteren seien Verbindungen der Formeln (Ib) und (Ic) 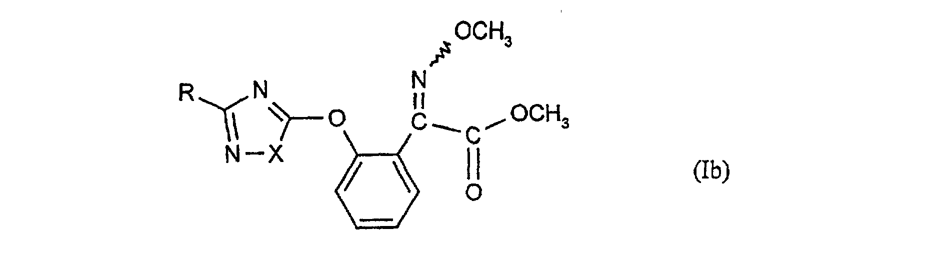 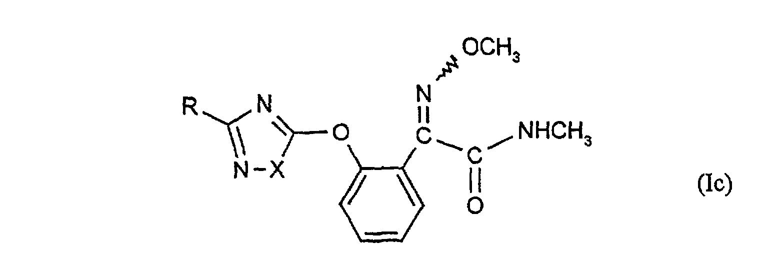 genannt,
in denen X für Schwefel steht und R die in Tabelle 1 angegebenen Bedeutungen hat sowie

Verbindungen der Formeln (Ib) und (Ic), in denen X für Sauerstoff steht und R die in Tabelle 1 angegebenen Bedeutungen hat.

Verwendet man beispielsweise 2-(2-Hydroxyphenyl)-3-methoxy-acrylsäuremethylester und 5-Chlor-3-phenoxy-1,2,4-thiadiazol als Ausgangsstoffe, so läßt sich der Reaktionsablauf des erfindungsgemäßen Verfahrens durch das folgende Formelschema darstellen:

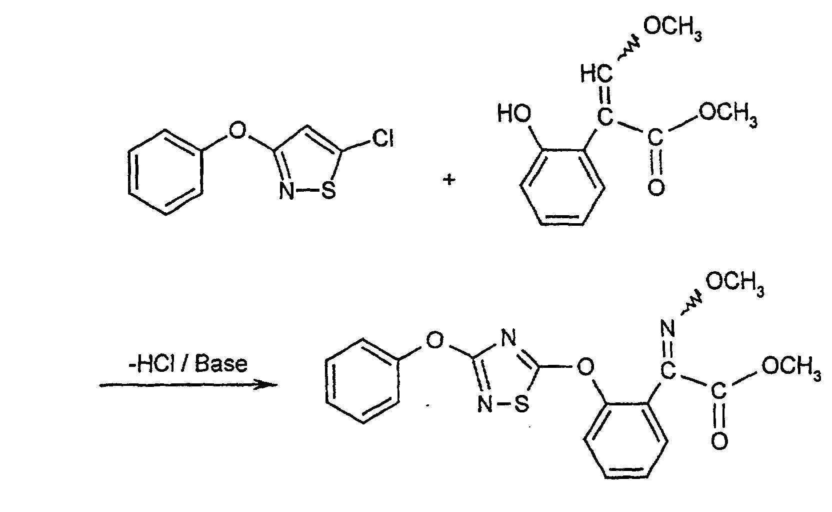

Die zur Durchführung des erfindungsgemäßen Verfahrens als Ausgangsverbindungen benötigten 2-Hydroxyphenylacrylate sind durch die Formel (II) definiert. 2-Hydroxyphenylacrylate der Formel (II) sind bekannt (vergl. z.B. EP 242 081, EP 468 684 und EP 477 631).

Die zur Durchführung des erfindungsgemäßen Verfahrens weiterhin als Ausgangsstoffe benötigten Diazolderivate sind durch die Formel (III) allgemein definiert. In dieser Formel (III) steht R vorzugsweise für diejenigen Reste, die bereits im Zusammenhang mit der Beschreibung der erfindungsgemäßen Verbindungen der Formel (I) als bevorzugt für diesen Substituenten genannt wurden. E steht für einen elektronenanziehenden Abgangsrest, vorzugsweise für Halogen, insbesondere für Chlor, Brom oder Iod oder für jeweils gegebenenfalls substituiertes Alkylsulfonyloxy, Alkoxysulfonyloxy oder Arylsulfonyloxy, wie insbesondere Methansulfonyloxy, Trifluormethansulfonyloxy, Methoxysulfonyloxy, Ethoxysulfonyloxy oder p-Toluolsulfonyloxy.

Die Thiadiazolderivate der Formel (III) sind bekannt oder erhältlich in Analogie zu bekannten Verfahren (vergl. z.B. DE 22 42 187; DE 11 32 379), beispielsweise wenn man Amidin-Derivate der Formel (IV), 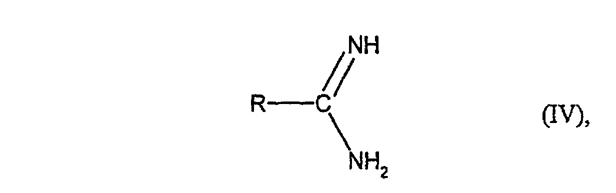 in welcher
- R: die oben angegebene Bedeutung hat,
oder deren Säureadditionssalze mit Trichlormethansulfenylchlorid gegebenenfalls in Gegenwart eines Verdünnungsmittels wie beispielsweise Dichlormethan und gegebenenfalls in Gegenwart eines Reaktionshilfsmittels wie beispielsweise Natriumhydroxid bei Temperaturen zwischen -20°C und +60°C umsetzt.

Amidin-Derivate der Formel (IV) sind allgemein bekannte Verbindungen der organischen Chemie oder erhältlich in Ananlogie zu bekannten Verfahren (vergl. z.B. EP 325 336).

Die Oxadiazolderivate der Formel (III) sind neu. Man erhält sie, indem man die Oxadiazolone der Formel (V) 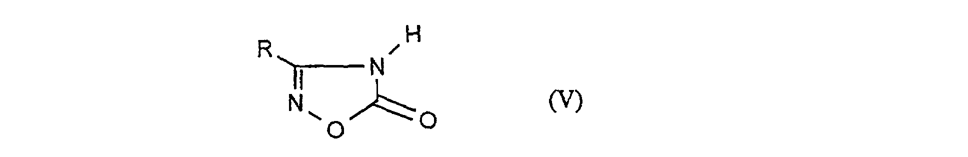 mit einem Chlorierungsmittel wie z.B. POCl₃ gegebenenfalls in Gegenwart eines geeigneten Lösungsmittels und gegebenenfalls in Gegenwart einer geeigneten Base umsetzt.

Besonders vorteilhaft ist es, die Verbindungen der Formel (V) mit POCl₃ in Pyridin umzusetzen.

Die Oxadiazolonderivate der Formel (V) sind bekannt oder erhältlich in Analogie zu bekannten Verfahren (vgl. z.B. Chem. Ber. 98, Seite 152, 153; Chem. Ber. 22, Seite 24).

Als Verdünnungsmittel zur Durchrührung des erfindungsgemäßen Verfahrens kommen inerte organische Lösungsmittel infrage. Hierzu gehören insbesondere aliphatische, alicyclische oder aromatische, gegebenenfalls halogenierte Kohlenwasserstoffe, wie beispielsweise Benzin, Benzol, Toluol, Xylol, Chlorbenzol, Dichlorbenzol, Petrolether, Hexan, Cyclohexan, Dichlormethan, Chloroform, Tetrachlorkohlenstoff; Ether, wie Diethylether, Diisopropylether, Dioxan, Tetrahydrofuran oder Ethylenglykoldimethyl- oder -diethylether; Nitrile, wie Acetonitril, Propionitril oder Benzonitril; Amide, wie N,N-Dimethylformamid, N,N-Dimethylacetamid, N-Methylformanilid, N-Methylpyrrolidon oder Hexamethylphosphorsäuretriamid oder Sulfoxide, wie Dimethylsulfoxid.

Das erfindungsgemäße Verfahren kann gegebenenfalls auch in einem Zweiphasensystem, wie beispielsweise Wasser/Toluol oder Wasser/Dichlormethan, gegebenenfalls in Gegenwart eines geeigneten Phasentransferkatalysators, durchgeführt werden. Als Beispiele für solche Katalysatoren seien genannt: Tetrabutylammoniumiodid, Tetrabutylammoniumbromid, Tetrabutylammoniumchlorid, Tributylmethylphosphoniumbromid, Trimethyl-C₁₃/C₁₅-alkylammoniumchlorid, Trimethyl-C₁₃/C₁₅-alkylammoniumbromid, Dibenzyldimethylammoniummethylsulfat, Dimethyl-C₁₂/C₁₄-alkylbenzylammoniumchlorid, Dimethyl-C₁₂/C₁₄-alkylbenzylammoniumbromid, Tetrabutylammoniumhydroxid, Triethylbenzylammoniumchlorid, Methyltrioctylammoniumchlorid, Trimethylbenzylammoniumchlorid, 15-Krone-5, 18-Krone-6 oder Tris-[2-(2-methoxyethoxy)-ethyl]-amin.

Das erfindungsgemäße Verfahren wird vorzugsweise in Gegenwart eines geeigneten Reaktionshilfsmittels durchgeführt. Als solche kommen alle üblichen anorganischen oder organischen Basen infrage. Hierzu gehören beispielsweise Erdalkali- oder Alkalimetallhydride, -hydroxide, -amide, -alkoholate, -acetate, -carbonate oder -hydrogencarbonate, wie beispielsweise Natriumhydrid, Natriumamid, Natriummethylat, Natriumethylat, Kalium-tert.-butylat, Natriumhydroxid, Kaliumhydroxid, Ammoniumhydroxid, Natriumacetat, Kaliumacetat, Calciumacetat, Ammoniumacetat, Natriumcarbonat, Kaliumcarbonat, Kaliumhydrogencarbonat, Natriumhydrogencarbonat oder Ammoniumcarbonat sowie tertiäre Amine, wie Trimethylamin, Triethylamin, Tributylamin, N,N-Dimethylanilin, Pyridin, N-Methylpiperidin, N,N-Dimethylaminopyridin, Diazabicyclooctan (DABCO), Diazabicyclononen (DBN) oder Diazabicycloundecen (DBU).

Die Reaktionstemperaturen können bei der Durchführung des erfindungsgemäßen Verfahrens in einem größeren Bereich variiert werden. Im allgemeinen arbeitet man bei Temperaturen zwischen -30°C und +120°C, vorzugsweise bei Temperaturen zwischen -20°C und +60°C.

Das erfindungsgemäße Verfahren wird üblicherweise unter Normaldruck durchgeführt. Es ist jedoch auch möglich unter erhöhtem oder vermindertem Druck zu arbeiten.

Zur Durchführung des erfindungsgemäßen Verfahrens setzt man pro Mol an 2-Hydroxyphenylacrylat der Formel (II) im allgemeinen 1,0 bis 10,0 Mol, vorzugsweise 1,0 bis 5,0 Mol an Diazolderivat der Formel (III) und gegebenenfalls 1,0 bis 5,0 Mol, vorzugsweise 1,0 bis 2,5 Mol an Reaktionshilfsmittel ein. Die Reaktionsdurchführung, Aufarbeitung und Isolierung der Reaktionsprodukte erfolgt nach bekannten Verfahren (vergl. hierzu auch die Herstellungsbeispiele).

Die Reinigung der Endprodukte der Formel (I) erfolgt mit Hilfe üblicher Verfahren, beispielsweise durch Säulenchromatographie oder durch Umkristallisieren.

Die Charakterisierung erfolgt mit Hilfe des Schmelzpunktes oder bei nicht kristallisierenden Verbindungen mit Hilfe des Brechungsindex oder der Protonen-Kernresonanzspektroskopie (¹H-NMR).

Die erfindungsgemäßen Wirkstoffe weisen eine starke mikrobizide Wirkung auf und können zur Bekämpfung von unerwünschten Mikroorganismen praktisch eingesetzt werden. Die Wirkstoffe sind für den Gebrauch als Pflanzenschutzmittel, insbesondere als Fungizide geeignet.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Beispielhaft aber nicht begrenzend seien einige Erreger von pilzlichen Krankheiten, die unter die oben aufgezählten Oberbegriffe fallen, genannt:
Pythium-Arten, wie beispielsweise Pythium ultimum;
Phytophthora-Arten, wie beispielsweise Phytophthora infestans;
Pseudoperonospora-Arten, wie beispielsweise Pseudoperonospora humuli oder Pseudoperonospora cubense;
Plasmopara-Arten, wie beispielsweise Plasmopara viticola;
Peronospora-Arten, wie beispielsweise Peronospora pisi oder Peronospora brassicae;
Erysiphe-Arten, wie beispielsweise Erysiphe graminis;
Sphaerotheca-Arten, wie beispielsweise Sphaerotheca fuliginea;
Podosphaera-Arten, wie beispielsweise Podosphaera leucotricha;
Venturia-Arten, wie beispielsweise Venturia inaequalis;
Pyrenophora-Arten, wie beispielweise Pyrenophora teres oder Pyrenophora graminea (Konidienform: Drechslera, Synonym: Helminthosporium);
Cochliobolus-Arten, wie beispielsweise Cochliobolus sativus (Konidienform: Drechslera, Synonym: Helminthosporium);
Uromyces-Arten, wie beispielsweise Uromyces appendiculatus;
Puccinia-Arten, wie beispielsweise Puccinia recondita;
Tilletia-Arten, wie beispielsweise Tilletia caries;
Ustilago-Arten, wie beispielsweise Ustilago nuda oder Ustilago avenae;
Pellicularia-Arten, wie beispielsweise Pellicularia sasakii;
Pyricularia-Arten, wie beispielsweise Pyricularia oryzae;
Fusarium-Arten, wie beispielsweise Fusarium culmorum;
Botrytis-Arten, wie beispielsweise Botrytis cinerea;
Septoria-Arten, wie beispielsweise Septoria nodorum;
Leptosphaeria-Arten, wie beispielsweise Leptosphaeria nodorum;
Cercospora-Arten, wie beispielsweise Cercospora canescens;
Alternaria-Arten, wie beispielsweise Alternaria brassicae;
Pseudocercosporella-Arten, wie beispielsweise Pseudocercosporella herpotrichoides.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut und des Bodens.

Dabei können die erfindungsgemäßen Wirkstoffe mit besonders gutem Erfolg zur Bekämpfung von Getreidekrankheiten, wie beispielsweise gegen den Erreger des echten Getreidemehltaues an Weizen oder Gerste (Erysiphe graminis) oder gegen den Erreger der Braunspelzigkeit des Weizens (Septoria nodorum bzw. Leptosphaeria nodorum) oder gegen den Erreger der Netzflecken-Krankheit der Gerste (Pyrenophora teres) oder gegen Fusaium-Arten oder zur Bekämpfung von Krankheiten im Obst- und Gemüseanbau, wie beispielsweise gegen den Erreger des echten Rebenmehltaues (Plasmopara viticola) oder gegen den Erreger des Apfelschorfes (Venturia inaequalis) oder gegen Phytophthoraerkrankungen an Tomaten oder zur Bekämpfung von Reiskrankheiten, wie beispielsweise gegen den Erreger der Reisfleckenkrankheit (Pyricularia oryzae) eingesetzt werden. Daneben besitzen die erfindungsgemäßen Wirkstoffe eine gute in vitro-Wirksamkeit.

Die Wirkstoffe können in Abhängigkeit von ihren jeweiligen physikalischen und/oder chemischen Eigenschaften in übliche Formulierungen übergeführt werden, wie Lösungen, Emulsionen, Suspensionen, Pulver, Schäume, Pasten, Granulate, Aerosole, Feinstverkapselungen in polymeren Stoffen und in Hüllmassen für Saatgut, sowie ULV-Kalt- und -Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen infrage: Aromaten, wie Xylol, Toluol, Alkylnaphthaline, chlorierte Aromaten oder chlorierte aliphatische Kohlenwasserstoffe, wie Chlorbenzole, Chlorethylene, oder Methylenchlorid, aliphatsche Kohlenwasserstoffe, wie Cyclohexan oder Paraffine, z.B. Erdölfraktionen, Alkohole, wie Butanol oder Glykol sowie deren Ether und Ester, Ketone, wie Aceton, Methylethylketon, Methylisobutylketon oder Cyclohexanon, stark polare Lösungsmittel, wie Dimethylformamid oder Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid; als feste Trägerstoffe kommen infrage: z.B. natürliche Gesteinsmehle, wie Kaoline, Tonerden, Talkum, Kreide, Quartz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen infrage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier- und/oder schaumerzeugende Mittel kommen infrage: z.B. nicht ionogene und anionische Emulgatoren, wie Polyoxyethylen-Fettsäureester, Polyoxyethylen-Fettalkohol-Ether, z.B. Alkylarylpolyglykolether, Alkylsulfonate, Alkylsulfate, Arylsulfonate sowie Eiweißhydrolysate; als Dispergiermittel kommen infrage: z.B. Ligninsulfitablaugen und Methylcellulose.

Es können in den Formulierungen Haftmittel wie Carboxymethylcellulose, natürliche und synthetische, pulverige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinenzwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Fungiziden, Bakteriziden, Akariziden, Nematiziden oder Insektiziden verwendet werden, um so z.B. das Wirkungsspektrum zu verbreitern oder Resistenzentwicklungen vorzubeugen. In vielen Fällen treten dabei synergistische Effekte auf.

Für die Mischungen kommen beispielsweise infrage:

### Fungizide:

2-Aminobutan; 2-Anilino-4-methyl-6-cyclopropyl-pyrimidin; 2',6'-Dibromo-2-methyl-4'-trifluoromethoxy-4'-trifluoro-methyl-1,3-thiazol-5-carboxanilid; 2,6-Dichloro-N-(4-trifluoromethylbenzyl)-benzamid; (E)-2-Methoxyimino-N-methyl-2-(2-phenoxyphenyl)-acetamid; 8-Hydroxyquinolinsulfat; Methyl-(E)-2-{2-[6-(2-cyanophenoxy)-pyrimidin-4-yloxy]-phenyl}-3-methoxyacrylat; Methyl-(E)-methoximino-[alpha-(o-tolyloxy)-o-tolyl]acetat; 2-Phenylphenol (OPP), Aldimorph, Ampropylfos, Anilazin, Azaconazol,
Benalaxyl, Benodanil, Benomyl, Binapacryl, Biphenyl, Bitertanol, Blasticidin-S, Bromuconazole, Bupirimate, Buthiobate,
Calciumpolysulfid, Captafol, Captan, Carbendazim, Carboxin, Chinomethionat (Quinomethionat), Chloroneb, Chloropicrin, Chlorothalonil, Chlozolinat, Cufraneb, Cymoxanil, Cyproconazole, Cyprofuram,
Dichlorophen, Diclobutrazol, Diclofluanid, Diclomezin, Dicloran, Diethofencarb, Difenoconazol, Dimethirimol, Dimethomorph, Diniconazol, Dinocap, Diphenylamin, Dipyrithion, Ditalimfos, Dithianon, Dodine, Drazoxolon,
Edifenphos, Epoxyconazole, Ethirimol, Etridiazol,
Fenarimol, Fenbuconazole, Fenfuram, Fenitropan, Fenpiclonil, Fenpropidin, Fenpropimorph, Fentinacetat, Fentinhydroxyd, Ferbam, Ferimzone, Fluazinam, Fludioxonil, Fluoromide, Fluquinconazole, Flusilazole, Flusulfamide, Flutolanil, Flutriafol, Folpet, Fosetyl-Aluminium, Fthalide, Fuberidazol, Furalaxyl, Furmecyclox, Guazatine,
Hexachlorobenzol, Hexaconazol, Hymexazol,
Imazalil, Imibenconazol, Iminoctadin, Iprobenfos (IBP), Iprodion, Isoprothiolan,
Kasugamycin, Kupfer-Zubereitungen, wie: Kupferhydroxid, Kupfernaphthenat, Kupferoxychlorid, Kupfersulfat, Kupferoxid, Oxin-Kupfer und Bordeaux-Mischung,
Mancopper, Mancozeb, Maneb, Mepanipyrim, Mepronil, Metalaxyl, Metconazol, Methasulfocarb, Methfuroxam, Metiram, Metsulfovax, Myclobutanil,
Nickel-dimethyldithiocarbamat, Nitrothal-isopropyl, Nuarimol,
Ofurace, Oxadixyl, Oxamocarb, Oxycarboxin,
Pefurazoat, Penconazol, Pencycuron, Phosdiphen, Phthalid, Pimaricin, Piperalin, Polycarbamate, Polyoxin, Probenazol, Prochloraz, Procymidon, Propamocarb, Propiconazole, Propineb, Pyrazophos, Pyrifenox, Pyrimethanil, Pyroquilon,
Quintozen (PCNB),
Schwefel und Schwefel-Zubereitungen,
Tebuconazol, Tecloftalam, Tecnazen, Tetraconazol, Thiabendazol, Thicyofen, Thiophanat-methyl, Thiram, Tolclophos-methyl, Tolylfluanid, Triadimefon, Triadimenol, Triazoxid, Trichlamid, Tricyclazol, Tridemorph, Triflumizol, Triforin, Triticonazol,
Validamycin A, Vinclozolin,
Zineb, Ziram.

### Bakterizide:

Bronopol, Dichlorophen, Nitrapyrin, Nickel-Dimethyldithiocarbamat, Kasugamycin, Octhilinon, Furancarbonsäure, Oxytetracyclin, Probenazol, Streptomycin, Tecloftalam, Kupfersulfat und andere Kupfer-Zubereitungen.

### Insektizide / Akarizide / Nematizide:

Abamectin, AC 303 630, Acephat, Acrinathrin, Alanycarb, Aldicarb, Alphamethrin, Amitraz, Avermectin, AZ 60541, Azadirachtin, Azinphos A, Azinphos M, Azocyclotin,
Bacillus thuringiensis, Bendiocarb, Benfuracarb, Bensultap, Betacyfluthrin, Bifenthrin, BPMC, Brofenprox, Bromophos A, Bufencarb, Buprofezin, Butocarboxim, Butylpyridaben,
Cadusafos, Carbaryl, Carbofuran, Carbophenothion, Carbosulfan, Cartap, CGA 157 419, CGA 184699, Chloethocarb, Chlorethoxyfos, Chlorfenvinphos, Chlorfluazuron, Chlormephos, Chlorpyrifos, Chlorpyrifos M, Cis-Resmethrin, Clocythrin, Clofentezin, Cyanophos, Cycloprothrin, Cyfluthrin, Cyhalothrin, Cyhexatin, Cypermethrin, Cyromazin,
Deltamethrin, Demeton M, Demeton S, Demeton-S-methyl, Diafenthiuron, Diazinon, Dichlofenthion, Dichlorvos, Dicliphos, Dicrotophos, Diethion, Diflubenzuron, Dimethoat, Dimethylvinphos, Dioxathion, Disulfoton,
Edifenphos, Emamectin, Esfenvalerat, Ethiofencarb, Ethion, Ethofenprox, Ethoprophos, Etrimphos,
Fenamiphos, Fenazaquin, Fenbutatinoxid, Fenitrothion, Fenobucarb, Fenothiocarb, Fenoxycarb, Fenpropathrin, Fenpyrad, Fenpyroximat, Fenthion, Fenvalerate, Fipronil, Fluazinam, Flucycloxuron, Flucythrinat, Flufenoxuron, Flufenprox, Fluvalinate, Fonophos, Formothion, Fosthiazat, Fubfenprox, Furathiocarb,
HCH, Heptenophos, Hexaflumuron, Hexythiazox,
Imidacloprid, Iprobenfos, Isazophos, Isofenphos, Isoprocarb, Isoxathion, Ivermectin, Lambda-cyhaloirin, Lufenuron,
Malathion, Mecarbam, Mevinphos, Mesulfenphos, Metaldehyd, Methacrifos, Methamidophos, Methidathion, Methiocarb, Methomyl, Metolcarb, Milbemectin, Monocrotophos, Moxidectin,
Naled, NC 184, NI 25, Nitenpyram,
Omethoat, Oxamyl, Oxydemethon M, Oxydeprofos,
Parathion A, Parathion M, Permethrin, Phenthoat, Phorat, Phosalon, Phosmet, Phosphamidon, Phoxim, Pirimicarb, Pirimiphos M, Pirimiphos A, Profenofos, Promecarb, Propaphos, Propoxur, Prothiofos, Prothoat, Pymetrozin, Pyrachlophos, Pyridaphenthion, Pyresmethrin, Pyrethrum, Pyridaben, Pyrimidifen, Pyriproxifen,
Quinalphos,
RH 5992,
Salithion, Sebufos, Silafluofen, Sulfotep, Sulprofos,
Tebufenozid, Tebufenpyrad, Tebupirimiphos, Teflubenzuron, Tefluthrin, Temephos, Terbam, Terbufos, Tetrachlorvinphos, Thiafenox, Thiodicarb, Thiofanox, Thiomethon, Thionazin, Thuringiensin, Tralomethrin, Triarathen, Triazophos, Triazuron, Trichlorfon, Triflumuron, Trimethacarb,
Vamidothion, XMC, Xylylcarb, YI 5301/5302, Zetamethrin.

Auch eine Mischung mit anderen bekannten Wirkstoffen, wie Herbiziden oder mit Düngemitteln und Wachstumsregulatoren ist möglich.

Die Wirkstoffe können als solche, in Form ihrer Formulierungen oder den daraus bereiteten Anwendungsformen, wie gebrauchsfertige Lösungen, Suspensionen, Spritzpulver, Pasten, lösliche Pulver, Stäubemittel und Granulate angewendet werden. Die Anwendung geschieht in üblicher Weise, z.B. durch Gießen, Verspritzen, Versprühen, Verstreuen, Verstäuben, Verschäumen, Bestreichen usw. Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut der Pflanzen behandelt werden.

Bei der Behandlung von Pflanzenteilen können die Wirkstoffkonzentrationen in den Anwendungsformen in einem größeren Bereich variiert werden: Sie liegen im allgemeinen zwischen 1 und 0,0001 Gew.-%, vorzugsweise zwischen 0,5 und 0,001 Gew.-%.

Bei der Saatgutbehandlung werden im allgemeinen Wirkstoffmengen von 0,001 bis 50 g je Kilogramm Saatgut, vorzugsweise 0,01 bis 10 g benötigt.

Bei der Behandlung des Bodens sind Wirkstoffkonzentrationen von 0,00001 bis 0,1 Gew.-%, vorzugsweise von 0,0001 bis 0,02 Gew.-% am Wirkungsort erforderlich.

### Herstellungsbeispiele:

### Beispiel 1:

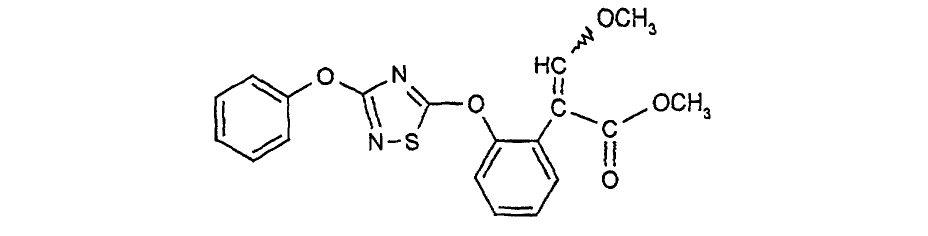

Zu einer Mischung von 1,6 g (0,008 Mol) 3-Phenoxy-5-chlor-1,2,4-thiadiazol und 1,6 g (0,008 Mol) 2-Hydroxyphenyl-3-methoxyacrylsäuremethylester (vergl. z.B. EP 242 081) in 50 ml absolutem Dimethylformamid gibt man bei -20°C 0,25 g (0,009 mol) Natriumhydrid (80%ig in Parrafinöl) und läßt den Reaktionsansatz anschließend langsam auf Raumtemperatur kommen. Zur Aufarbeitung gibt man unter Eiskühlung 15 ml gesättigte wässrige Natriumhydrogencarbonatlösung zu, extrahiert dreimal mit jeweils 20 ml Ether, säuert dann die wässrige Phase mit verdünnter Salzsäure an und extrahiert dreimal mit jeweils 20 ml Essigester. Die vereinigten organischen Phasen werden getrocknet und im Vakuum eingeengt und der Rückstand durch Chromatographie an Kieselgel (Laufmittel: Dichlormethan/Essigester 100:1) gereinigt.

Man erhält 1,8 g (62 % der Theorie) an 2-[2-(3-Phenoxy-1,2,4-thiadiazol-5-yloxy)-phenyl]-3-methoxy-acrylsäuremethylester als Öl.
¹H-NMR (CDCl₃/Tetramethylsilan): δ = 3,67 (s, 3H); 3,80 (s, 3H); 7,25 (m, 4H); 7,4 (m, 5H) 7,6 (s, 1H) ppm.

### Beispiel 2:

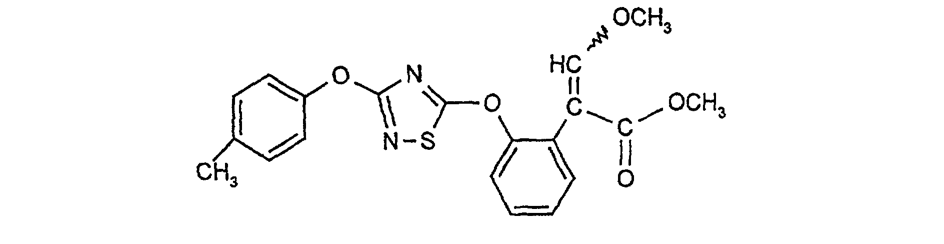

Zu einer Mischung von 10,5 g (0,05 Mol) 3-(4'-MePhenoxy)-5-chlor-1,2,4-oxadiazol und 10,4 g (0,05 Mol) 2-Hydroxyphenyl-3-methoxyacrylsäuremethylester (vergl. z.B. EP 242 081) in 50 ml absolutem Dimethylformamid gibt man bei -20°C 1,5 g (0,05 Mol) Natriumhydrid (80%ig in Parrafinöl) und läßt den Reaktionsansatz anschließend langsam auf Raumtemperatur kommen. Zur Aufarbeitung gießt man auch Wasser und extrahiert dreimal mit jeweils 20 ml Ether. Die vereinigten organischen Phasen werden getrocknet und im Vakuum eingeengt und der Rückstand durch Chromatographie an Kieselgel (Laufmittel: Dichlormethan) gereinigt.
Man erhält 10,5 g (55 % der Theorie) an 2-[2-(3-{4'-Me-Phenoxy}-1,2,4-oxadiazol-5-yloxy)-phenyl]-3-methoxy-acrylsäuremethylester als Öl.
¹H-NMR(CDCl₃/Tetramethylsilan): δ = 2,34 (3H); 3,68 (3H); 3,79 (3H); 7,1-7,5 (8H) 7,59 (1H) ppm.

### Herstellung der Ausgangsverbindung:

### Beispiel III-1:

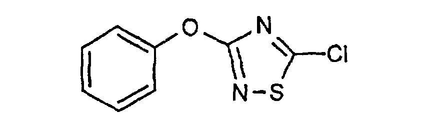

Zu 2,9 g (0,017 Mol) Phenoxy-formamidin Hydrochlorid (vergl. z.B. EP 325 336) in 30 ml Dichlormethan gibt man bei Raumtemperatur unter Rühren zunächst 1,6 ml (0,0151 Mol) Trichlormethansulfenylchlorid und anschließend bei -8°C tropfenweise unter Rühren und Eiskühlung 7,5 g ( 0,084 Mol) 45 prozentige wässrige Natronlauge. Danach läßt man die Reaktionsmischung auf Raumtemperatur erwärmen und rührt weitere 2 Stunden bei dieser Temperatur. Zur Aufarbeitung gibt man 16 g trockenes Natriumsulfat zu, rührt 10 Minuten, filtriert dann den Reaktionsansatz über Kieselgur, wäscht mit Dichlormethan nach und engt das Filtrat im Vakuum ein.

Man erhält 2,1 g (62 % der Theorie) an 3-Phenoxy-5-chlor-1,2,4-thiadiazol vom Schmelzpunkt 171°C.

### Beispiel III-2:

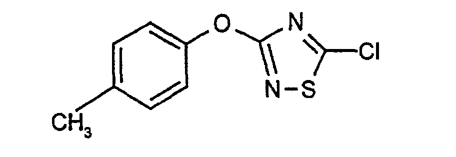

28,8 g (0,15 Mol) 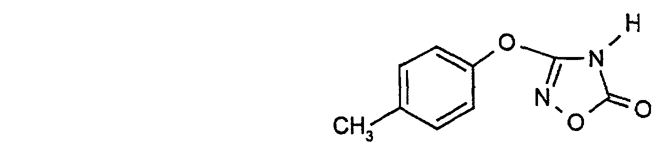 (vgl. Chem. Ber. 98, Seite 152, 153) werden in 28 ml POCl₃ eingetragen und mit 11,9 g (0,15 Mol) Pyridin versetzt. Man kocht 4 Stunden am Rückfluß, zieht das überschüssige POCl₃ ab, gießt auf Eiswasser und extrahiert mit Dichlormethan. Man engt ein, versetzt mit Diethylether, filtriert von Ungelöstem ab und engt ein. Man erhält 22 g (69,7 % der Theorie) Produkt.

Massespektrum: 214, 210, 175, 147, 107, 91, 77, 39.

In entsprechender Weise und gemäß den allgemeinen Angaben zur Herstellung erhält man die folgenden 3-Methoxy-2-phenyl-acrylsäureester der allgemeinen Formel

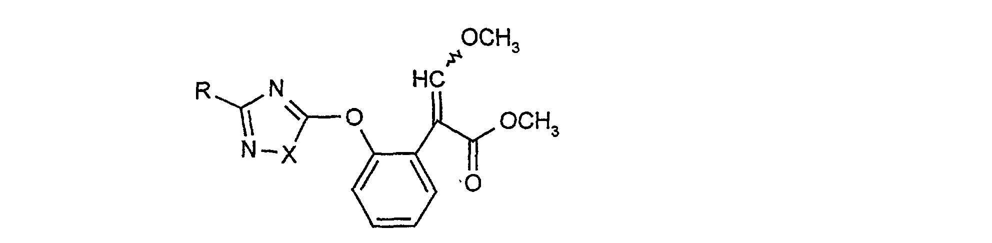

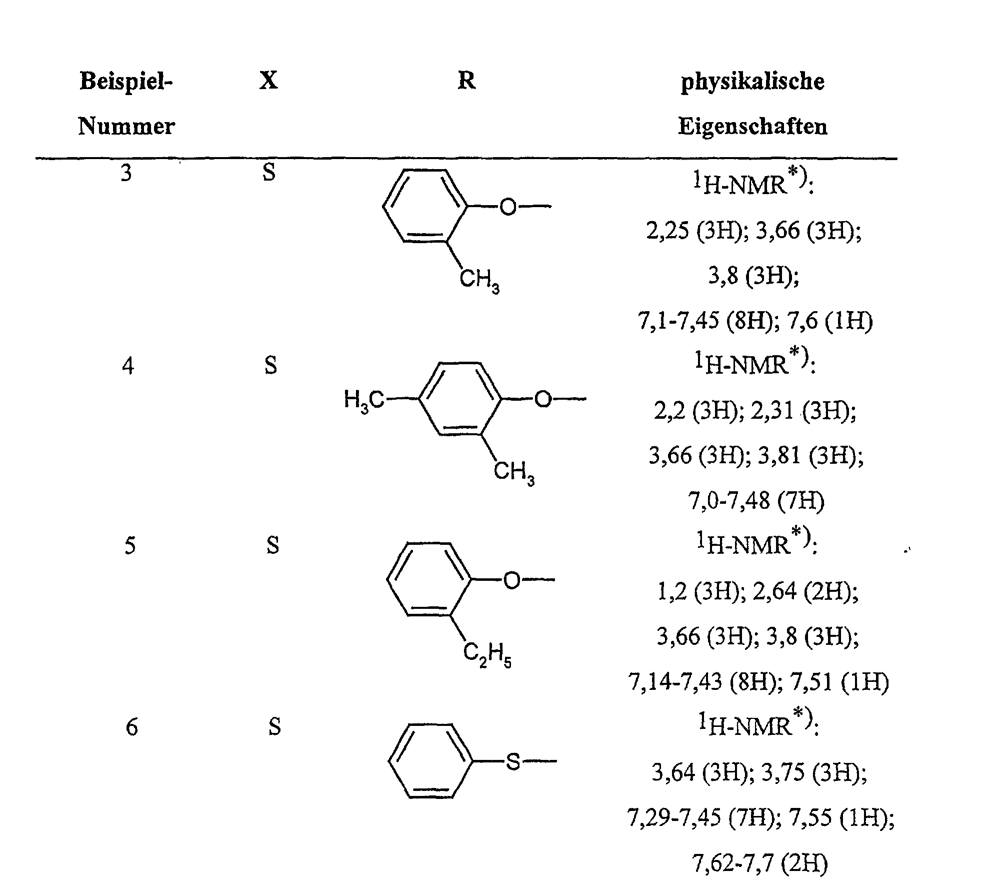

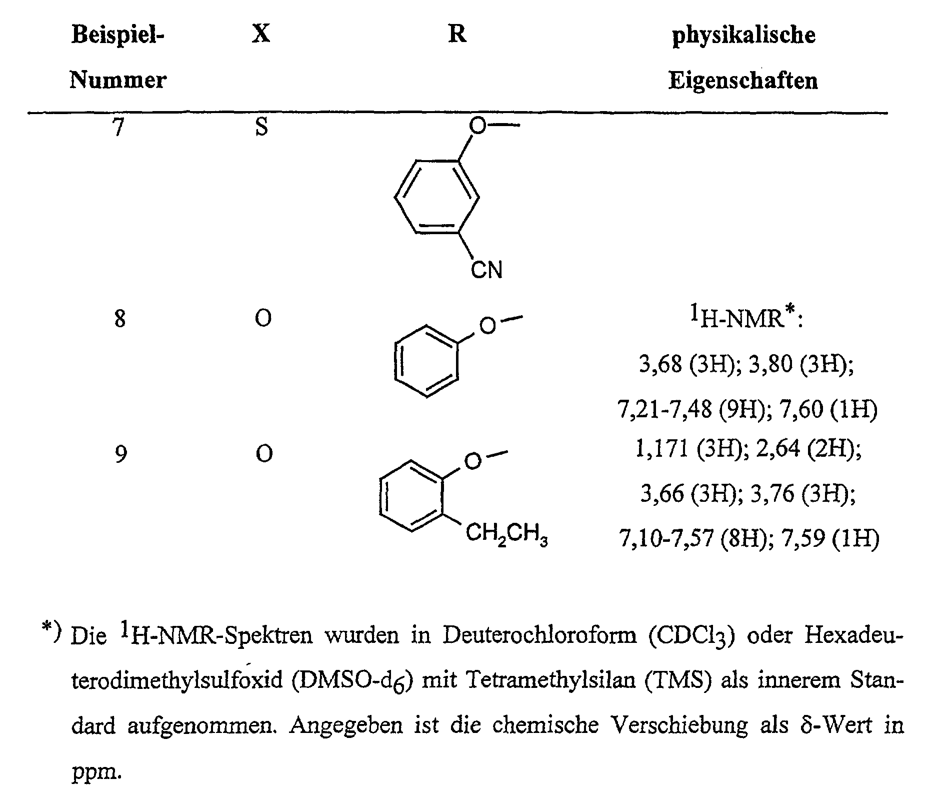

In entsprechender Weise und gemäß den allgemeinen Angaben zur Herstellung erhält man die folgenden Verbindungen der allgemeinen Formel (I).

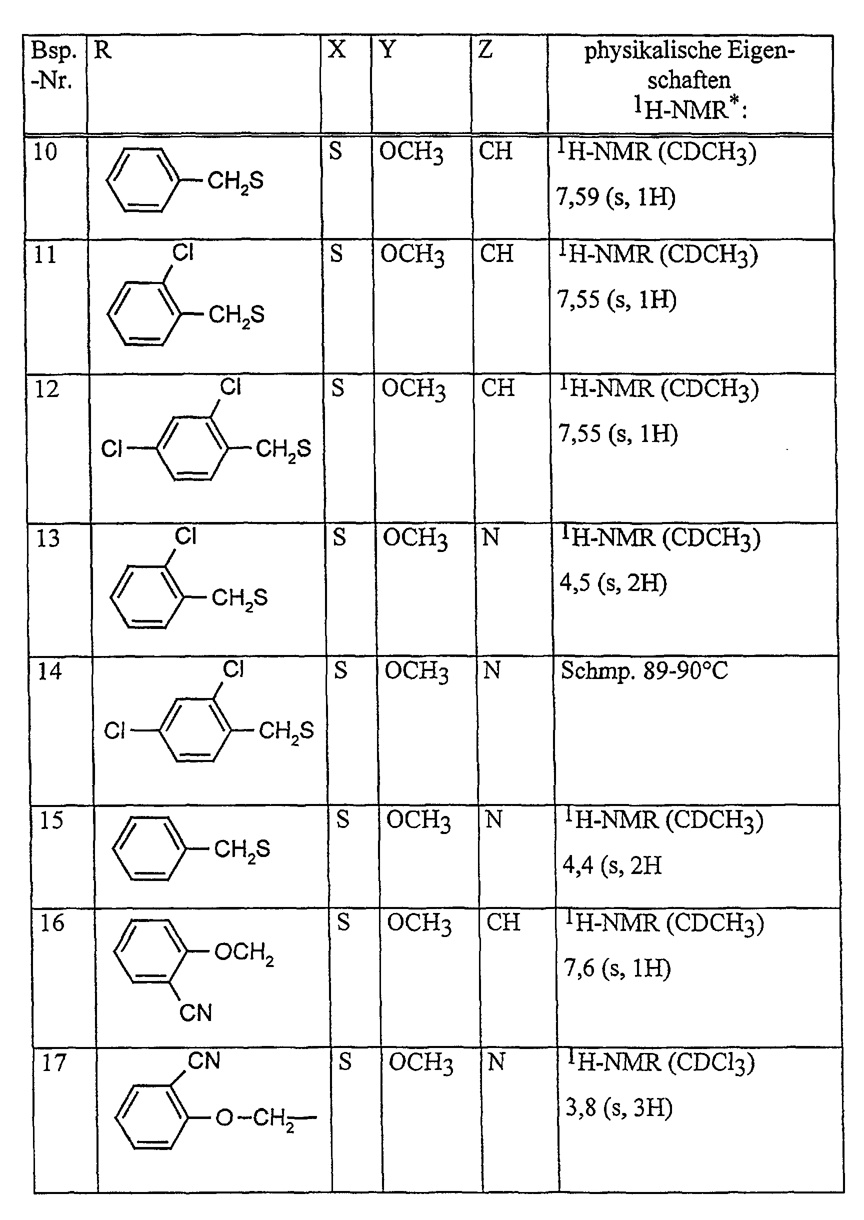

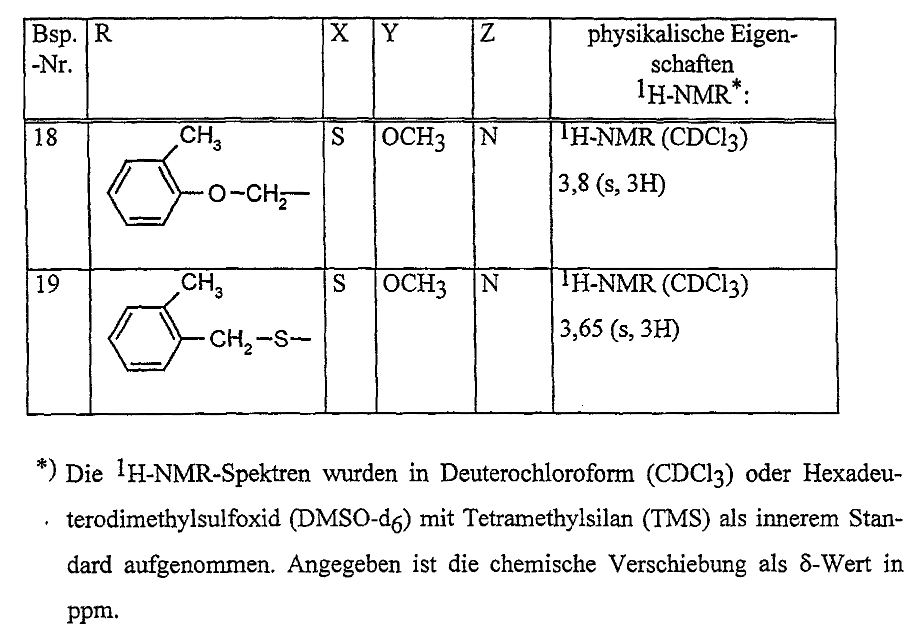

### Anwendungsbeispiele:

In den folgenden Anwendungsbeispielen wurde die nachstehend aufgeführte Verbindung als Vergleichssubstanz eingesetzt: 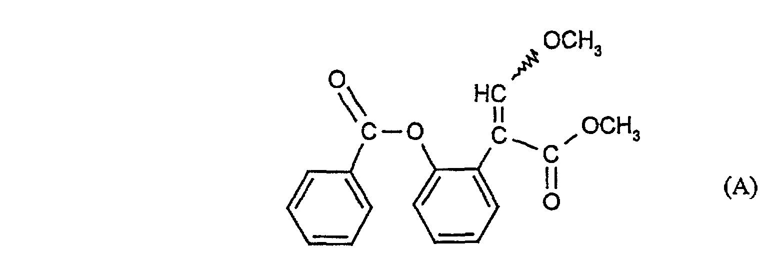 3-Methoxy-2-(2-benzoyloxyphenyl)-acrylsäureester (bekannt aus EP 178 826)

### Beispiel A:

### Venturia-Test (Apfel) / protektiv

- Lösungsmittel:: 4,7 Gewichtsteile Aceton
- Emulgator:: 0,3 Gewichtsteile Alkyl-Aryl-Polyglykolether

Zur Herstellung einer zweckmäßigen Wirkstoffzubereitung vermischt man ein Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden die Pflanzen mit einer wässrigen Konidiensuspension des Apfelschorferregers (Venturia inaequalis) inokuliert und verbleiben dann einen Tag bei 20°C und 100 % relativer Luftfeuchtigkeit in einer Inkubationskabine.

Die Pflanzen werden dann im Gewächshaus bei 20°C und einer relativen Luftfeuchtigkeit von ca. 70 % aufgestellt.

12 Tage nach der Inokulation erfolgt die Auswertung.

Eine deutliche Überlegenheit in der Wirksamkeit gegenüber dem Stand der Technik zeigen in diesem Test z.B. die Verbindungen gemäß der Herstellungsbeispiele 2 und 9, die bei einer Wirkstoffkonzentration von 10 ppm einen bis zu100 %igen Wirkungsgrad zeigen.

### Beispiel B:

### Plasmopara-Test (Rebe) / protektiv

- Lösungsmittel:: 4,7 Gewichtsteile Aceton
- Emulgator:: 0,3 Gewichtsteile Alkyl-Aryl-Polyglykolether

Zur Herstellung einer zweckmäßigen Wirkstoffzubereitung vermischt man ein Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden die Pflanzen mit einer wässrigen Sporensuspension von Plasmopara viticola inokuliert und verbleiben dann einen Tag in einer Feuchtkammer bei 20°C bis 22°C und 100% relativer Luftfeuchtigkeit. Anschließend werden die Pflanzen 5 Tage im Gewächshaus bei 21°C und 90% Luftfeuchtigkeit aufgestellt. Die Pflanzen werden dann angefeuchtet und einen Tag in eine Feuchtkammer gestellt.

6 Tage nach der Inokulation erfolgt die Auswertung.

Eine deutliche Überlegenheit in der Wirksamkeit gegenüber dem Stand der Technik zeigen in diesem Test z.B. die Verbindungen gemäß der Herstellungsbeispiele: 2, 8 und 9, die bei einer Wirkstoffkonzentration von 10 ppm einen Wirkungsgrad von 70 bis 100 % zeigen.

### Beispiel C:

### Phytophthora-Test (Tomate) / protektiv

- Lösungsmittel:: 4,7 Gewichtsteile Aceton
- Emulgator:: 0,3 Gewichtsteile Alkyl-Aryl-Polyglykolether

Zur Herstellung einer zweckmäßigen Wirkstoffzubereitung vermischt man ein Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstofizubereitung bis zur Tropfnässe. Nach Antrocknen des Spritzbelages werden die Pflanzen mit einer wäßrigen Sporensuspension von Phytophthora infestans inokuliert.

Die Pflanzen werden in einer Inkubationskabine mit 100% relativer Luftfeuchtigkeit und 20°C aufgestellt.

3 Tage nach der Inokulation erfolgt die Auswertung.

Eine deutliche Überlegenheit in der Wirksamkeit gegenüber dem Stand der Technik zeigen in diesem Test z.B. die Verbindung gemäß dem Herstellungsbeispiel: 2, welches bei einer Wirkstoffkonzentration von 10 ppm einen bis zu 100 %igen Wirkungsgrad zeigt.

### Beispiel D:

### Cochliobolus sativus-Test (Gerste) / protektiv

- Lösungsmittel:: 10 Gewichtsteile N-Methyl-pyrrolidon
- Emulgator:: 0,6 Gewichtsteile Alkyl-Aryl-Polyglykolether

Zur Herstellung einer zweckmäßigen Wirkstoffzubereitung vermischt man ein Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung taufeucht. Nach Antrocknen des Spritzbelages werden die Pflanzen mit einer Konidiensuspension von Cochliobolus sativus besprüht. Die Pflanzen verbleiben 48 Stunden bei 20°C und 100 % relativer Luftfeuchtigkeit in einer Inkubationskabine.

Die Pflanzen werden in einem Gewächshaus bei einer Temperatur von ca. 20°C und einer relativen Luftfeuchtigkeit von ca. 80 % aufgestellt.

7 Tage nach der Inokulation erfolgt die Auswertung.

Eine deutliche Überlegenheit in der Wirksamkeit gegenüber dem Stand der Technik zeigen bei diesem Test z.B. die Verbindungen gemäß dem Herstellungsbeispiel: 2, welches bei einer Wirkstoffkonzentration von 100 ppm einen 100 %-igen Wirkungsgrad zeigt.

## Patentansprüche

1. Verbindungen der Formel (I) 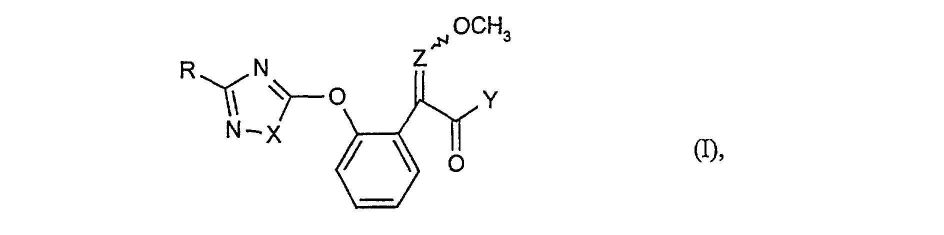 in welcher
Y für OCH₃ oder NHCH₃ steht,
Z für =CH- oder =N- steht,
R für Fluor, Chlor, Brom, Methyl, Ethyl, n-, i-, s-, t-Butyl, C₁-C₂-Halogenalkyl mit 1 bis 5 gleichen oder verschiedenen Halogenatomen oder für einen Rest der Formel Ar-A- steht, wobei
Ar für gegebenenfalls einfach bis fünffach, gleich oder verschieden substituiertes Aryl mit 6 oder 10 Kohlenstoffatomen oder für gegebenenfalls einfach bis vierfach, gleich oder verschieden substituiertes und/oder benzoannelliertes Heteroaryl mit 2 bis 9 Kohlenstoffatomen und 1 bis 3 gleichen oder verschieden Heteroatomen - Stickstoff, Sauerstoff und/oder Schwefel - steht, wobei als Substituenten jeweils infrage kommen:
Halogen, Cyano, Nitro, Hydroxy, Amino, Formyl, Carbamoyl, Thiocarbamoyl, jeweils geradkettiges oder verzweigtes Alkyl, Alkoxy, Alkylthio, Alkylsulfinyl oder Alkylsulfonyl mit jeweils 1 bis 4 Kohlenstoffatomen, jeweils geradkettiges oder verzweigtes Alkenyl oder Alkenyloxy mit jeweils 2 bis 4 Kohlenstoffatomen, jeweils geradkettiges oder verzweigtes Halogenalkyl, Halogenalkoxy, Halogenalkylthio, Halogenalkylsulfinyl oder Halogenalkylsulfonyl mit jeweils 1 bis 4 Kohlenstoffatomen und 1 bis 9 gleichen oder verschiedenen Halogenatomen, jeweils geradkettiges oder verzweigtes Halogenalkenyl oder Halogenalkenyloxy mit jeweils 2 bis 4 Kohlenstoffatomen und 1 bis 9 gleichen oder verschiedenen Halogenatomen, jeweils geradkettiges oder verzweigtes N-Alkylamino, Dialkylamino, Alkylcarbonyl, Alkylcarbonyloxy, Alkoxycarbonyl, Alkylsulfonyloxy, Hydroximinoalkyl oder Alkoximinoalkyl mit jeweils 1 bis 4 Kohlenstoffatomen in den einzelnen Alkyl- und Alkoxyteilen, jeweils gegebenenfalls einfach oder mehrfach, gleich oder verschieden durch Halogen und/oder geradkettiges oder verzweigtes Alkyl mit 1 bis 3 Kohlenstoffatomen und/oder geradkettiges oder verzweigtes Halogenalkyl mit 1 bis 3 Kohlenstoffatomen und 1 bis 7 gleichen oder verschiedenen Halogenatomen substituiertes, jeweils zweifach verknüpftes Alkylen oder Dioxyalkylen mit jeweils 1 bis 4 Kohlenstoffatomen, Cycloalkyl mit 3 bis 6 Kohlenstoffatomen, 5- bis 7-gliedriges, gesättigtes Heterocyclyl mit 4 bis 6 Kohlenstoffatomen und 1 oder 2 gleichen oder verschiedenen Heteroatomen - Stickstoff, Sauerstoff und/oder Schwefel -, sowie jeweils gegebenenfalls im Phenylteil einfach bis dreifach, gleich oder verschieden durch Halogen und/oder geradkettiges oder verzweigtes Alkyl mit 1 bis 3 Kohlenstoffatomen und/oder geradkettiges oder verzweigtes Halogenalkyl mit 1 bis 3 Kohlenstoffatomen und 1 bis 7 gleichen oder verschiedenen Halogenatomen und/oder geradkettiges oder verzweigtes Alkoxy mit 1 bis 3 Kohlenstoffatomen und/oder geradkettiges oder verzweigtes Halogenalkoxy mit 1 bis 3 Kohlenstoffatomen und 1 bis 7 gleichen oder verschiedenen Halogenatomen substituiertes Phenyl, Phenoxy, Benzyl, Benzyloxy, Phenylethyl oder Phenylethyloxy und
A für Sauerstoff, Schwefel oder eine Gruppierung der Formel -S(O)-, -SO₂-, -NH-, -N(CH₃)-, -CH₂O-, -CH₂S-, -OCH₂- oder -SCH₂- steht, und
X für Sauerstoff oder Schwefel steht,
ausgenommen Verbindungen, in denen
R für Fluor, Chlor, Brom, Methyl, Ethyl, n-, i-, s-, t-Butyl, C₁-C₄-Halogenalkyl mit 1 bis 5 gleichen oder verschiedenen Halogenatomen und
X für S steht.

2. Verbindungen der Formel (I), gemäß Anspruch 1 bei welchen
R für Chlor, Brom, Methyl, Ethyl, CF₃, CH₂F, CHF₂, CCl₃, CH₂Cl, CHCl₂, CFCl₂, CF₂Cl oder für einen Rest der Formel Ar-A- steht, wobei
Ar für jeweils gegebenenfalls einfach bis dreifach, gleich oder verschieden substituiertes Phenyl oder Naphthyl oder für jeweils gegebenenfalls einfach bis dreifach, gleich oder verschieden substituiertes und/oder benzoannelliertes Furanyl, Thienyl, Pyrrolyl, Oxazolyl, Thiazolyl, Isoxazolyl, Isothiazolyl, Pyrazolyl, Imidazolyl, Oxadiazolyl, Thiadiazolyl, Triazolyl, Pyridyl, Pyridazinyl, Pyrimidinyl, Pyrazinyl oder Triazinyl steht, wobei als Substituenten jeweils infrage kommen:
Fluor, Chlor, Brom, Hydroxy, Cyano, Nitro, Amino, Formyl, Carbamoyl, Thiocarbamoyl, Methyl, Ethyl, n- oder i-Propyl, n-, i-, s- oder t-Butyl, Methoxy, Ethoxy, n- oder i-Propoxy, n-, i-, s- oder t-Butoxy, Methylthio, Ethylthio, Methylsulfinyl, Methylsulfonyl, Allyl, Butenyl, Allyloxy, Butenyloxy, Trifluormethyl, Trifluormethoxy, Trifluormethylthio, Trifluormethylsulfinyl, Trifluormethylsulfonyl, Dimethylamino, Diethylamino, Acetyl, Acetoxy, Methylsulfonyloxy, Ethylsulfonyloxy, Methoxycarbonyl, Ethoxycarbonyl, Hydroximinomethyl, Hydroximinoethyl, Methoximinomethyl, Methoximinoethyl, Ethoximinomethyl, Ethoximinoethyl, Propan-1,3-diyl, Butan-1,4-diyl, Dioxyethylen, Dioxyethylen, Dioxypropylen, Difluordioxymethylen, Tetrafluordioxyethylen, Cyclopropyl, Cyclopentyl, Cyclohexyl, 1-Pyrrolidinyl, 1-Piperidinyl, 1-Perhydroazepinyl, 4-Morpholinyl oder jeweils gegebenenfalls ein- bis dreifach, gleich oder verschieden durch Fluor, Chlor, Brom, Methyl, Ethyl, Methoxy, Ethoxy, Trifluormethyl und/oder Trifluormethoxy substituiertes Phenyl, Phenoxy, Benzyl oder Benzyloxy und
A für Sauerstoff, Schwefel oder eine Gruppierung der Formel -S(O)-, -SO₂-, -NH-, -N(CH₃)-, -CH₂O-, -CH₂S-, -OCH₂- oder -SCH₂- steht, und
X für Sauerstoff oder Schwefel steht,
ausgenommen Verbindungen, in denen
R für Chlor, Brom, Methyl, Ethyl, CF₃, CH₂F, CHF₂, CCl₃, CH₂Cl, CHCl₂, CFCl₂, CF₂Cl und
X für S steht.

3. Schädlingsbekämpfungsmittel, gekennzeichnet durch einen Gehalt an mindestens einer Verbindung der Formel (I) nach den Ansprüchen 1 bis 2.

4. Verfahren zur Bekämpfung von Schädlingen, dadurch gekennzeichnet, daß man Verbindungen der Formel (I) nach den Ansprüchen 1 bis 2 auf Schädlinge und/oder ihren Lebensraum einwirken läßt.

5. Verwendung von Verbindungen der Formel (I) nach den Ansprüchen 1-2 zur Bekämpfung von Schädlingen.

6. Verfahren zur Herstellung von Oxa(Thia)-diazol-oxy-phenylacrylaten der allgemeinen Formel (I), 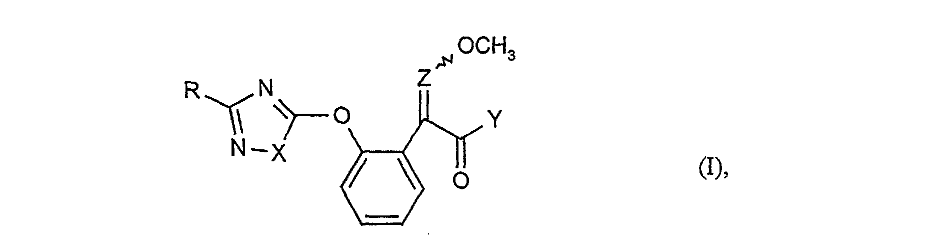 in welcher
Y für OCH₃ oder NHCH₃ steht,
Z für =CH- oder =N- steht, und
R, Ar, A und X die in Anspruch 1 angegebene Bedeutung haben.
dadurch gekennzeichnet, daß man 2-Hydroxyphenylacrylate der Formel (II) 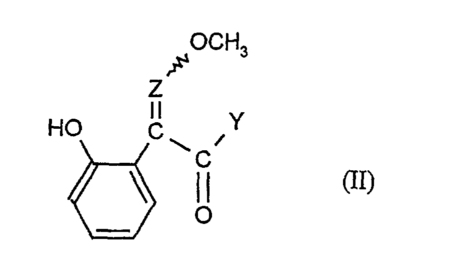 mit Oxa- bzw. Thiadiazolderivaten der Formel (III), 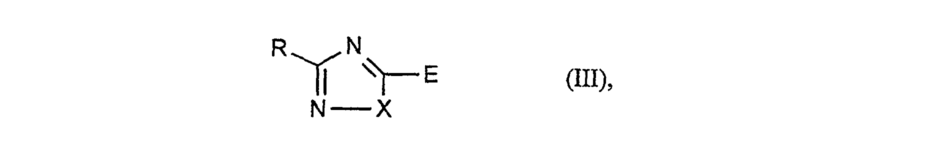 in welcher
R und X die oben angegebenen Bedeutungen haben und
E für eine elektronenanziehende Abgangsgruppe steht,
gegebenenfalls in Gegenwart eines Verdünnungsmittels und gegebenenfalls in Gegenwart eines Reaktionshilfsmittels umsetzt.

7. Verfahren zur Herstellung von Schädlingsbekämpfungsmitteln, dadurch gekennzeichnet, daß man Verbindungen der Formel (I) nach den Ansprüchen 1 bis 2 mit Streckmitteln und/oder oberflächenaktiven Mitteln vermischt.

8. Oxadiazolderivate der Formel (IIIa) 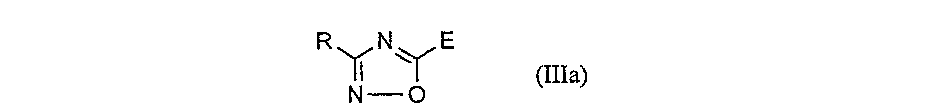 in welcher
R die in Anspruch 1 angegebenen Bedeutungen hat und
E für eine elektronenanziehende Abgangsgruppe steht.

## Claims

1. Compounds of the formula (I), 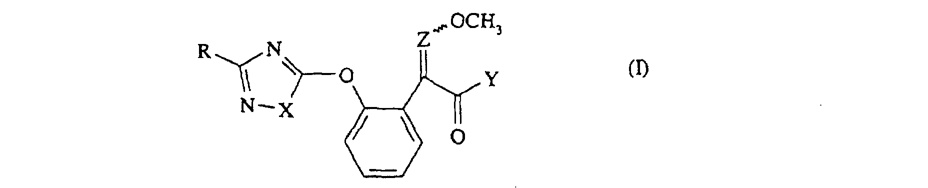 in which
Y represents OCH₃ or NHCH₃,
Z represents =CH- or =N-,
R represents fluorine, chlorine, bromine, methyl, ethyl, n-, i-, s- or t-butyl, C₁-C₂-halogenoalkyl having 1 to 5 identical or different halogen atoms, or represents a radical of the formula Ar-A-, where
Ar represents aryl having 6 or 10 carbon atoms which is optionally substituted identically or differently once to five times, or represents heteroaryl having 2 to 9 carbon atoms and 1 to 3 identical or different hetero atoms - nitrogen, oxygen and/or sulphur - which is optionally substituted, identically or differently once to four times, and/or benzo-fused, with suitable substituents in each case being:
halogen, cyano, nitro, hydroxyl, amino, formyl, carbamoyl, thiocarbamoyl, in each case straight-chain or branched alkyl, alkoxy, alkylthio, alkylsulphinyl or alkylsulphonyl having in each case 1 to 4 carbon atoms, in each case straight-chain or branched alkenyl or alkenyloxy having in each case 2 to 4 carbon atoms, in each case straight-chain or branched halogenoalkyl, halogenoalkoxy, halogenoalkylthio, halogenoalkylsulphinyl or halogenoalkylsulphonyl having in each case 1 to 4 carbon atoms and 1 to 9 identical or different halogen atoms, in each case straight-chain or branched halogenoalkenyl or halogenoalkenyloxy having in each case 2 to 4 carbon atoms and 1 to 9 identical or different halogen atoms, in each case straight-chain or branched N-alkylamino, dialkylamino, alkylcarbonyl, alkylcarbonyloxy, alkoxycarbonyl, alkylsulphonyloxy, hydroximinoalkyl or alkoximinoalkyl having in each case 1 to 4 carbon atoms in the individual alkyl and alkoxy moieties, in each case doubly linked alkylene or dioxyalkylene having in each case 1 to 4 carbon atoms which is in each case optionally substituted identically or differently once or more than once by halogen and/or straight-chain or branched alkyl having 1 to 3 carbon atoms and/or straight-chain or branched halogenoalkyl having 1 to 3 carbon atoms and 1 to 7 identical or different halogen atoms, cycloalkyl having 3 to 6 carbon atoms, 5- to 7-membered saturated heterocyclyl having 4 to 6 carbon atoms and 1 or 2 identical or different hetero atoms - nitrogen, oxygen and/or sulphur -, as well as phenyl, phenoxy, benzyl, benzyloxy, phenylethyl or phenylethyloxy which is in each case optionally substituted identically or differently once to three times in the phenyl moiety by halogen and/or straight-chain or branched alkyl having 1 to 3 carbon atoms and/or straight-chain or branched halogenoalkyl having 1 to 3 carbon atoms and 1 to 7 identical or different halogen atoms and/or straight-chain or branched alkoxy having 1 to 3 carbon atoms and/or straight-chain or branched halogenoalkoxy having 1 to 3 carbon atoms and 1 to 7 identical or different halogen atoms, and
A represents oxygen, sulphur, or a group of the formula -S(O)-, -SO₂-, -NH-, -N(CH₃)-, -CH₂O-, -CH₂S-, -OCH₂- or -SCH₂-, and
X represents oxygen or sulphur,
excluding compounds in which
R represents fluorine, chlorine, bromine, methyl, ethyl, n-, i-, s- or t-butyl, C₁-C₄-halogenoalkyl having 1 to 5 identical or different halogen atoms, and
X represents S.

2. Compounds of the formula (I), according to Claim 1, in which
R represents chlorine, bromine, methyl, ethyl, CF₃, CH₂F, CHF₂, CCl₃, CH₂Cl, CHCl₂, CFCl₂ or CF₂Cl, or represents a radical of the formula Ar-A-, where
Ar represents phenyl or naphthyl which is in each case optionally substituted identically or differently once to three times, or represents furanyl, thienyl, pyrrolyl, oxazolyl, thiazolyl, isoxazolyl, isothiazolyl, pyrazolyl, imidazolyl, oxadiazolyl, thiadiazolyl, triazolyl, pyridyl, pyridazinyl, pyrimidinyl, pyrazinyl or triazinyl which is in each case optionally substituted, identically or differently once to three times, and/or benzo-fused, with suitable substituents in each case being:
fluorine, chlorine, bromine, hydroxyl, cyano, nitro, amino, formyl, carbamoyl, thiocarbamoyl, methyl, ethyl, n- or i-propyl, n-, i-, s- or t-butyl, methoxy, ethoxy, n- or i-propoxy, n-, i-, s- or t-butoxy, methylthio, ethylthio, methylsulphinyl, methylsulphonyl, allyl, butenyl, allyloxy, butenyloxy, trifluoromethyl, trifluoromethoxy, trifluoromethylthio, trifluoromethylsulphinyl, trifluoromethylsulphonyl, dimethylamino, diethylamino, acetyl, acetoxy, methylsulphonyloxy, ethylsulphonyloxy, methoxycarbonyl, ethoxycarbonyl, hydroximinomethyl, hydroximinoethyl, methoximinomethyl, methoximinoethyl, ethoximinomethyl, ethoximinoethyl, propane-1,3-diyl, butane-1,4-diyl, dioxymethylene, dioxyethylene, dioxypropylene, difluorodioxymethylene, tetrafluorodioxyethylene, cyclopropyl, cyclopentyl, cyclohexyl, 1-pyrrolidinyl, 1-piperidinyl, 1-perhydroazepinyl, 4-morpholinyl, or phenyl, phenoxy, benzyl or benzyloxy which is in each case optionally substituted identically or differently once to three times by fluorine, chlorine, bromine, methyl, ethyl, methoxy, ethoxy, trifluoromethyl and/or trifluoromethoxy, and
A represents oxygen, sulphur, or a group of the formula -S(O)-, -SO₂-, -NH-, -N(CH₃)-, -CH₂O-, -CH₂S-, -OCH₂- or -SCH₂-, and
X represents oxygen or sulphur,
excluding compounds in which
R represents chlorine, bromine, methyl, ethyl, CF₃, CH₂F, CHF₂, CCl₃, CH₂Cl, CHCl₂, CFCl₂ or CF₂Cl, and
X represents S.

3. Composition for controlling pests, characterized by a content of at least one compound of the formula (I) according to Claims 1 or 2.

4. Process for controlling pests, characterized in that compounds of the formula (I) according to Claims 1 or 2 are allowed to act on pests and/or their habitat.

5. Use of compounds of the formula (I) according to Claims 1-2 for controlling pests.

6. Process for preparing oxa(thia)-diazol-oxy-phenylacrylates of the general formula (I), 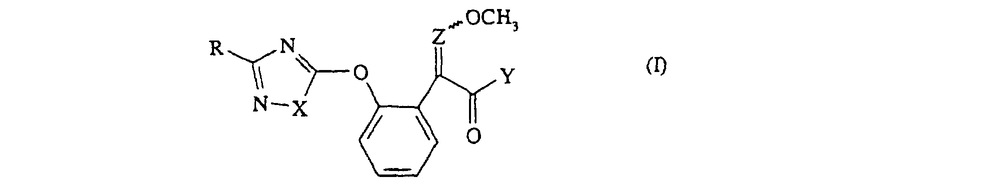 in which
Y represents OCH₃ or NHCH₃,
Z represents =CH- or =N-, and
R, Ar, A and X have the meanings given in Claim 1,
characterized in that 2-hydroxyphenylacrylates of the formula (II) 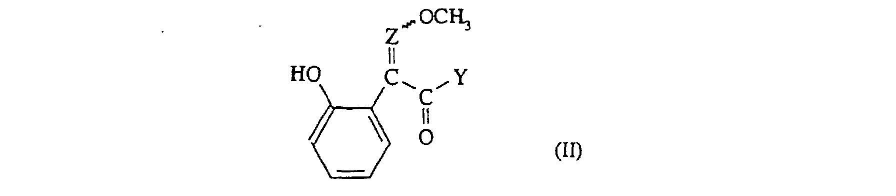 are reacted with oxadiazole or thiadiazole derivatives of the formula (III), 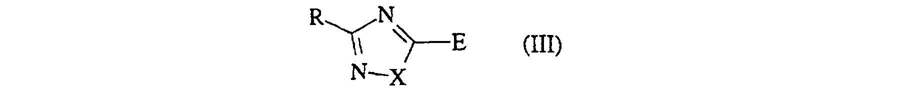 in which
R and X have the abovementioned meanings, and
E represents an electron-attracting leaving group,
optionally in the presence of a diluent and optionally in the presence of a reaction auxiliary.

7. Process for preparing agents for controlling pests, characterized in that compounds of the formula (I) according to Claims 1 or 2 are mixed with extenders and/or surface-active agents.

8. Oxadiazole derivatives of the formula (IIIa) 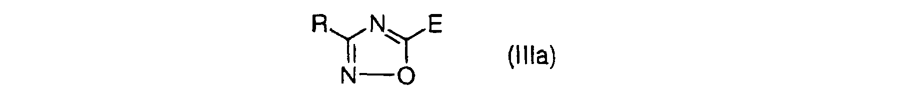 in which
R has the meanings given in Claim 1, and
E represents an electron-attracting leaving group.

## Revendications

1. Composés de formule (I) 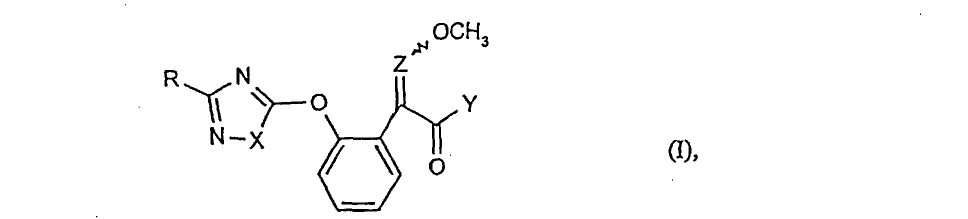 dans laquelle
Y représente OCH₃ ou NHCH₃,
Z représente =CH- ou =N-,
R représente le fluor, le chlore, le brome, un groupe méthyle, éthyle, n-butyle, isobutyle, sec.-butyle, tertio-butyle, halogénalkyle en C₁ ou C₂ portant 1 à 5 atomes d'halogènes identiques ou différents, ou un reste de formule Ar-A-, dans laquelle
Ar représente un groupe aryle de 6 ou 10 atomes de carbone éventuellement substitué 1 à 5 fois identiques ou différentes ou un groupe hétéroaryle de 2 à 9 atomes de carbone et 1 à 3 hétéroatomes identiques ou différents - azote, oxygène et/ou soufre - portant éventuellement 1 à 4 substituants identiques ou différents et/ou éventuellement condensé au benzène, les substituants considérés étant dans chaque cas :
halogène, cyano, nitro, hydroxy, amino, formyle, carbamoyle, thiocarbamoyle, alkyle, alkoxy, alkylthio, alkylsulfinyle ou alkylsulfonyle linéaire ou ramifié dans chaque cas et ayant chacun 1 à 4 atomes de carbone, alcényle ou alcényloxy linéaire ou ramifié dans chaque cas et ayant chacun 2 à 4 atomes de carbone, halogénalkyle, halogénalkoxy, halogénalkylthio, halogénalkylsulfinyle ou halogénalkylsulfonyle linéaire ou ramifié dans chaque cas et ayant chacun 1 à 4 atomes de carbone et 1 à 9 atomes d'halogènes identiques ou différents, halogénalcényle ou halogénalcényloxy linéaire ou ramifié dans chaque cas et ayant chacun 2 à 4 atomes de carbone et 1 à 9 atomes d'halogènes identiques ou différents, N-alkylamino, dialkylamino, alkylcarbonyle, alkylcarbonyloxy, alkoxycarbonyle, alkylsulfonyloxy, hydroximino-alkyle ou alkoximino-alkyle linéaire ou ramifié dans chaque cas et ayant chacun 1 à 4 atomes de carbone dans les parties alkyle et alkoxy individuelles, alkylène ou dioxy-alkylène ayant chacun deux liaisons et chacun 1 à 4 atomes de carbone et chacun étant éventuellement substitué une ou plusieurs fois identiques ou différentes par un halogène et/ou un substituant alkyle linéaire ou ramifié ayant 1 à 3 atomes de carbone et/ou halogénalkyle linéaire ou ramifié ayant 1 à 3 atomes de carbone et 1 à 7 atomes d'halogènes identiques ou différents, cycloalkyle ayant 3 à 6 atomes de carbone, hétérocyclyle saturé pentagonal à heptagonal ayant 4 à 6 atomes de carbone et 1 ou 2 hétéroatomes - azote, oxygène et/ou soufre - identiques ou différents, ainsi que phényle, phénoxy, benzyle, benzyloxy, phényléthyle ou phényléthyloxy portant chacun le cas échéant dans la partie phényle 1 à 3 substituants, identiques ou différents, halogène et/ou alkyle linéaire ou ramifié ayant 1 à 3 atomes de carbone et/ou halogénalkyle linéaire ou ramifié ayant 1 à 3 atomes de carbone et 1 à 7 atomes d'halogènes identiques ou différents et/ou alkoxy linéaire ou ramifié ayant 1 à 3 atomes de carbone et/ou halogénalkoxy linéaire ou ramifié ayant 1 à 3 atomes de carbone et 1 à 7 atomes d'halogènes identiques ou différents, et
A représente l'oxygène, le soufre ou un groupement de formule -S (O) -, -SO₂-, -NH-, -N(CH₃)-, -CH₂O-, -CH₂S-, -OCH₂- ou -SCH₂-, et
X représente l'oxygène ou le soufre,
excepté les composés dans lesquels
R représente le fluor, le chlore, le brome, un groupe méthyle, éthyle, n-butyle, isobutyle, sec.-butyle, tertio-butyle, halogénalkyle en C₁ à C₄ portant 1 à 5 atomes d'halogènes identiques ou différents et
X représente S.

2. Composés de formule (I) suivant la revendication 1, dans lesquels
R représente le chlore, le brome, un groupe méthyle, éthyle, CF₃, CH₂F, CHF₂, CCl₃, CH₂Cl, CHCl₂, CFCl₂, CF₂Cl ou un reste de formule Ar-A-, dans laquelle
Ar représente un groupe phényle ou naphtyle portant chacun le cas échéant 1 à 3 substituants identiques ou différents, ou un groupe furannyle, thiényle, pyrrolyle, oxazolyle, thiazolyle, isoxazolyle, isothiazolyle, pyrazolyle, imidazolyle, oxadiazolyle, thiadiazolyle, triazolyle, pyridyle, pyridazinyle, pyrimidinyle, pyrazinyle ou triazinyle dont chacun porte éventuellement 1 à 3 substituants identiques ou différents et/ou est éventuellement condensé au benzène, les substituants considérés étant dans chaque cas :
fluor, chlore, brome, hydroxy, cyano, nitro, amino, formyle, carbamoyle, thiocarbamoyle, méthyle, éthyle, n-propyle, isopropyle, n-butyle, isobutyle, sec.-butyle, tertio-butyle, méthoxy, éthoxy, n-propoxy, isopropoxy, n-butoxy, isobutoxy, sec.-butoxy, tertio-butoxy, méthylthio, éthylthio, méthylsulfinyle, méthylsulfonyle, allyle, butényle, allyloxy, butényloxy, trifluorométhyle, trifluorométhoxy, trifluorométhylthio, trifluorométhylsulfinyle, trifluorométhylsulfonyle, diméthylamino, diéthylamino, acétyle, acétoxy, méthylsulfonyloxy, éthylsulfonyloxy, méthoxy-carbonyle, éthoxy-carbonyle, hydroximinométhyle, hydroximino-éthyle, méthoximino-méthyle, méthoximino-éthyle, éthoximino-méthyle, éthoximino-éthyle, propane-1,3-diyle, butane-1,4-diyle, dioxyéthylène, dioxyéthylène, dioxypropylène, difluorodioxyméthylène, tétrafluorodioxyéthylène, cyclopropyle, cyclopentyle, cyclohexyle, 1-pyrrolidinyle, 1-pipéridinyle, 1-perhydroazépinyle, 4-morpholinyle, ou phényle, phénoxy, benzyle ou benzyloxy portant chacun le cas échéant 1 à 3 substituants fluoro, chloro, bromo, méthyle, éthyle, méthoxy, éthoxy, trifluorométhyle et/ou trifluorométhoxy identiques ou différents, et
A représente l'oxygène, le soufre ou un groupement de formule -S(O)-, -SO₂-, -NH-, -N(CH₃)-, -CH₂O-, -CH₂S-, -OCH₂- ou -SCH₂-, et
X représente l'oxygène ou le soufre,
excepté les composés dans lesquels
R représente le chlore, le brome, un groupe méthyle, éthyle, CF₃, CH₂F, CHF₂, CCl₃, CH₂Cl, CHCl₂, CFCl₂, CF₂Cl et
X représente S.

3. Composition pesticide, caractérisée par une teneur en au moins un composé de formule (I) suivant les revendications 1 et 2.

4. Procédé pour combattre des parasites, caractérisé en ce qu'on fait agir des composés de formule (I) suivant les revendications 1 et 2 sur les parasites et/ou sur leur milieu.

5. Utilisation de composés de formule (I) suivant les revendications 1 et 2 pour combattre des parasites.

6. Procédé de production d'acrylates d'oxa-(thia)-diazole-oxy-phényle de formule générale (I), 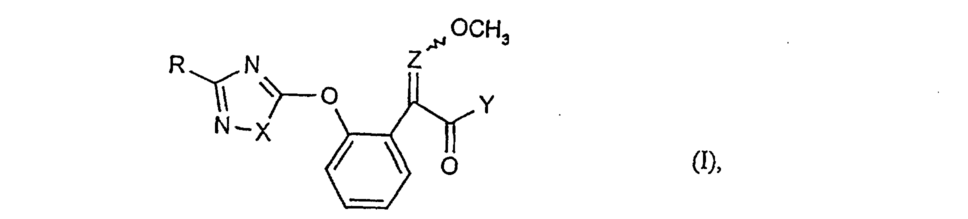 dans laquelle
Y représente OCH₃ ou NHCH₃,
Z représente =CH- ou =N-, et
R, Ar, A et X ont la définition indiquée dans la revendication 1,
caractérisé en ce qu'on fait réagir des acrylates de 2-hydroxyphényle de formule (II) 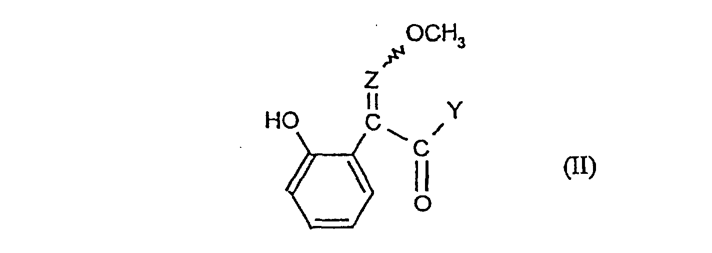 avec des dérivés d'oxa- ou de thiadiazole de formule (III), 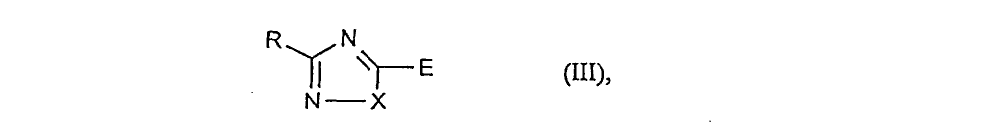 dans laquelle
R et X ont les définitions indiquées ci-dessus et
E représente un groupe partant attirant les électrons,
le cas échéant en présence d'un diluant et en présence éventuelle d'un auxiliaire de réaction.

7. Procédé de préparation de compositions pesticides, caractérisé en ce qu'on mélange des composés de formule (I) suivant les revendications 1 et 2 avec des diluants et/ou des agents tensio-actifs.

8. Dérivés d'oxadiazole de formule (IIIa) 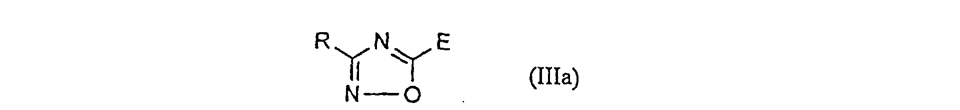 dans laquelle
R a les définitions indiquées dans la revendication 1 et
E est un groupe partant attirant les électrons.
